(19) Europäisches Patentamt
European Patent Office
Office européen des brevets

(11) Veröffentlichungsnummer: **0 451 585 A2**

(12) **EUROPÄISCHE PATENTANMELDUNG**

(21) Anmeldenummer: **91104607.6**

(22) Anmeldetag: **23.03.91**

(51) Int. Cl.[5]: **C07D 409/04**, C07D 405/04, C07D 401/04, C07D 417/04, C07D 413/04, C07D 213/68, C07D 213/71, C07D 213/70, C07D 213/74, A01N 43/40

(30) Priorität: **07.04.90 DE 4011361**

(43) Veröffentlichungstag der Anmeldung: **16.10.91 Patentblatt 91/42**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Yanagi, Akihiko, Dr.**
**2-1200-1, Nagabuchi, Oume-Chi**
**Tokyo(JP)**
Erfinder: **Heinemann, Ulrich, Dr.**
**Am Sonnenhang 1**
**W-5653 Leichlingen 2(DE)**
Erfinder: **Babczinski, Peter, Dr.**
**In der Lohrenbeck 11**
**W-5600 Wuppertal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leberkusen(DE)**
Erfinder: **Schmidt, Robert R., Dr.**
**Im Waldwinkel 110**
**W-5060 Bergisch Gladbach 2(DE)**

(54) **2-Aryl-6-hetarylpyridin-Derivate.**

(57) Neue 2-Aryl-6-hetarylpyridin-Derivate der allgemeinen Formel (I),

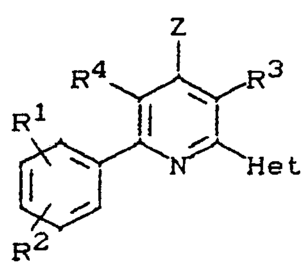

(I)

in welcher

Z für $X_n$-$R^{5-1}$ oder

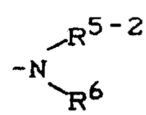

EP 0 451 585 A2

und

Het für gegebenenfalls substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht,

mehrere Verfahren sowie neue Zwischenprodukte zu deren Herstellung und ihre Verwendung als Herbizide.

Die Erfindung betrifft neue 2-Aryl-6-hetarylpyridin-Derivate, neue Zwischenprodukte sowie mehrere Verfahren zu ihrer Herstellung und ihre Verwendung als Herbizide.

Es ist bereits bekannt, daß bestimmte 4-substituierte 2,6-Diphenylpyridin-Derivate herbizide Eigenschaften besitzen (vergleiche EP-A 263 958).

Weiterhin bekannt sind folgende Verbindungen; 5-Methyl-3-trifluormethyl-1-(6-phenyl-4-trifluormethyl-2-pyridyl)-pyrazol (vergleiche J. Heterocycl. Chem., 6 (2), 223 (1969)γ; 2-(4-Dimethylamino-phenyl)-6-(2,5-dimethylpyrrol-1-yl)-pyridin (vergleiche J. Amer. Chem. Soc. 68. 326 (1946)); 6-Phenyl-(2,2'-bipyridyl) (vergleiche Chem. Ber. 109, 3864 (1976)); 4-Methylthio-6-phenyl-2,2'-bipyridin (vergleiche US-P 4 451 659); 4-Methyl-2-phenyl-6-(2-thienyl)-pyridin (vergleiche J. Org. Chem. 50 (7), 1125-6 (1985)); 6-[4-(Brommethyl)-phenyl]-2,2'-bipyridin; 6-(4-Methylphenyl)-2,2'-bipyridin (vergleiche Inorg. Chem. 22 (20), 2818-24 (1983)); 4-(Methylthio)-2-phenyl-6-(2-thienyl)-pyridin (vergleiche J. Org. Chem. 47 (16), 3027-38 (1982)).
Über die Verwendbarkeit der oben genannten Verbindungen als Herbizid ist jedoch nichts bekannt.

Es wurden neue 2-Aryl-6-hetarylpyridin-Derivate der allgemeinen Formel (I),

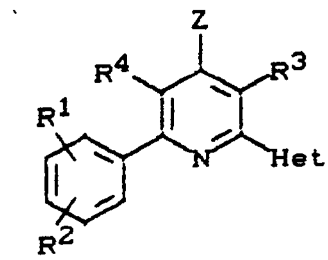

(I)

in welcher

$R^1$ für Wasserstoff, Halogen, Alkyl, Alkoxy oder Halogenalkyl steht,

$R^2$ für Wasserstoff, Halogen oder Halogenalkyl steht,

$R^3$ und $R^4$ unabhängig voneinander für Wasserstoff oder Alkyl stehen,

Het für jeweils unsubstituiertes oder substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht und

Z für Wasserstoff, Halogen oder die Gruppierungen $-X_n-R^{5-1}$ oder

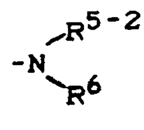

steht, wobei

X für Sauerstoff oder Schwefel steht,

$R^{5-1}$ und $R^{5-2}$ unabhängig voneinander für jeweils gegebenenfalls substituiertes Alkyl, Alkenyl oder Alkinyl steht,

$R^6$ für Wasserstoff oder Alkyl steht, oder

$R^{5-2}$ und $R^6$ gemeinsam mit dem Stickstoffatom, an welches sie gebunden sind, einen 5- bis 7-gliedrigen gesättigten oder ungesättigten Ring bilden, welcher gegebenenfalls ein weiteres Heteroatom enthält und welcher gegebenenfalls durch 1 oder 2 Alkylgruppen substituiert ist, und

n für 0 oder 1 steht,

ausgenommen die Verbindungen 5-Methyl-3-trifluormethyl-1-(6-phenyl-4-trifluormethyl-2-pyridyl-)pyrazol (vergleiche J. Heterocycl. Chem., 6 (2), 223 (1969)); 2-(4-Dimethylamino-phenyl)-6-(2,5-dimethyl-pyrrol-1-yl)-pyridin (vergleiche J. Amer. Chem. Soc. 68, 326 (1946)); 6-Phenyl-(2,2'-bipyridyl) (vergleiche Chem. Ber. 109, 3864 (1976)); 4-Methylthio-6-phenyl-2,2'-bipyridin (vergleiche US-P 4 451 659); 4-Methyl-2-phenyl-6-(2-thienyl)-pyridin (vergleiche J. Org. Chem. 50 (7), 1125-6 (1985)); 6-[4-(Brommethyl)phenyl]-2,2'-bipyridin; 6-(4-Methylphenyl)-2,2'-bipyridin (vergleiche Inorg. Chem. 22 (20), 2818-24 (1983)) und 4-(Methylthio)-2-phenyl-6-(2-thienyl)-pyridin (vergleiche J. Org. Chem. 47 (16), 3027-38 (1982)),
gefunden.

Weiterhin wurde gefunden, daß man die neuen 2-Aryl-6-hetarylpyridin-Derivateder Formel (I)

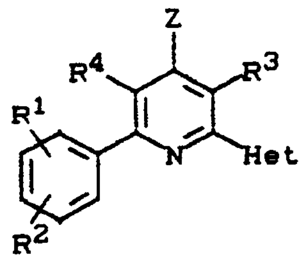

(I)

in welcher

| | |
|---|---|
| $R^1$ | für Wasserstoff, Halogen, Alkyl, Alkoxy oder Halogenalkyl steht, |
| $R^2$ | für Wasserstoff, Halogen oder Halogenalkyl steht, |
| $R^3$ und $R^4$ | unabhängig voneinander für Wasserstoff oder Alkyl stehen, |
| Het | für jeweils unsubstituiertes oder substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht und |
| Z | für Wasserstoff, Halogen oder die Gruppierungen $-X_n-R^{5-1}$ oder |

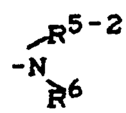

| | |
|---|---|
| | steht, wobei |
| X | für Sauerstoff oder Schwefel steht, |
| $R^{5-1}$ und $R^{5-2}$ | unabhängig voneinander für jeweils gegebenenfalls substituiertes Alkyl, Alkenyl oder Alkinyl steht, |
| $R^6$ | für Wasserstoff oder Alkyl steht, oder |
| $R^{5-2}$ und $R^6$ | gemeinsam mit dem Stickstoffatom, an welches sie gebunden sind, einen 5- bis 7-gliedrigen gesättigten oder ungesättigten Ring bilden, welcher gegebenenfalls ein weiteres Heteroatom enthält und welcher gegebenenfalls durch 1 oder 2 Alkylgruppen substituiert ist, und |
| n | für 0 oder 1 steht, |

ausgenommen die bereits oben genannten Verbindungen,
nach einem der im folgenden beschriebenen Verfahren erhält:

a) Man erhält die erfindungsgemäßen 2-Aryl-6-hetarylpyridin-Derivate dar Formel (Ia)

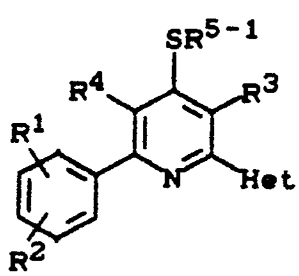

(Ia)

in welcher
$R^1$, $R^2$, $R^3$, $R^4$, $R^{5-1}$ und Hat die oben angegebene Bedeutung haben,
wenn man 1,5-Dioxo-Verbindungen der Formel (II) bzw. deren Isomere der Formel (IIa)

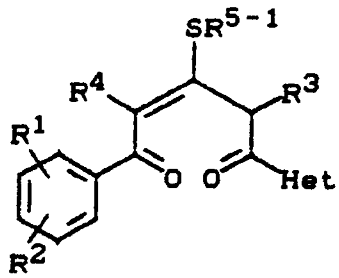

(II)

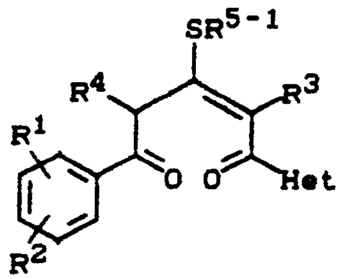

(IIa)

in welcher
$R^1$, $R^2$, $R^3$, $R^4$, $R^{5-1}$ und Het die oben angegebene Bedeutung haben,
mit Ammoniak oder einem Ammoniumsalz gegebenenfalls in Gegenwart eines Verdünnungsmittels umsetzt.

b) Man erhält die erfindungsgemäßen 2-Aryl-6-hetarylpyridin-Derivate der Formel (Ib)

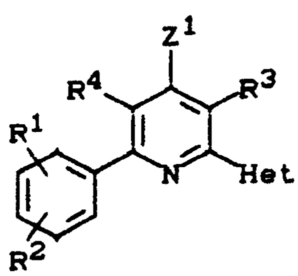

(Ib)

in welcher
$Z^1$ für $-OR^{5-1}$ oder

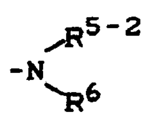

steht und
$R^1$, $R^2$, $R^3$, $R^4$, $R^{5-1}$, $R^{5-2}$, $R^6$ und Het die oben angegebene Bedeutung haben,
wenn man 4-Sulfonylalkyl-2-aryl-6-hetarylpyridin-Derivate der Formel (III)

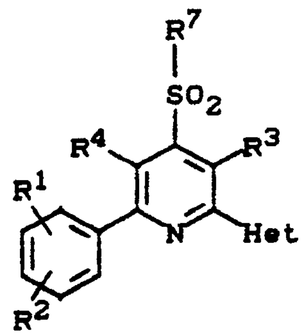

(III)

in welcher

$R^1$, $R^2$, $R^3$, $R^4$ und Het die oben angegebene Bedeutung haben und

$R^7$ für Alkyl steht, entweder

α) mit Verbindungen der Formel (IV)

$R^{5-1}$-OM (IV)

in welcher

$R^{5-1}$ die oben angegebene Bedeutung hat und

M für ein Alkalimetallkation steht,

gegebenenfalls in Gegenwart eines Verdünnungsmittels umsetzt, oder

β) mit Aminen der Formel (V)

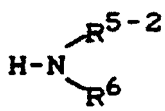

(V)

in welcher

$R^{5-2}$ und $R^6$ die oben angegebene Bedeutung haben,

gegebenenfalls in Gegenwart eines Verdünnungsmittels und gegebenenfalls unter erhöhtem Druck umsetzt.

c) Man erhält die erfindungsgemäßen 2-Aryl-6-hetarylpyridin-Derivate der Formel (Ic)

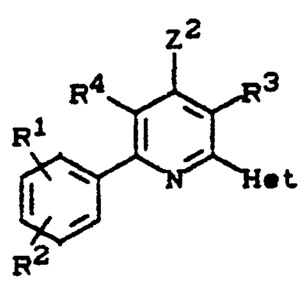

(Ic)

in welcher

$R^1$, $R^2$, $R^3$, $R^4$ und Het die oben angegebene Bedeutung haben und

$Z^2$ für Halogen steht,

wenn man 4-Hydroxy-2-aryl-6-hetarylpyridin-Derivate der Formel (VI)

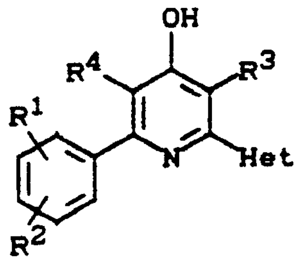

(VI)

in welcher

$R^1$, $R^2$, $R^3$, $R^4$ und Het die oben angegebene Bedeutung haben,

mit einem Halogenierungsreagenz gegebenenfalls in Gegenwart eines Verdünnungsmittels umsetzt.

d) Man erhält die erfindungsgemäßen 2-Aryl-6-hetarylpyridin-Derivate der Formel (Id)

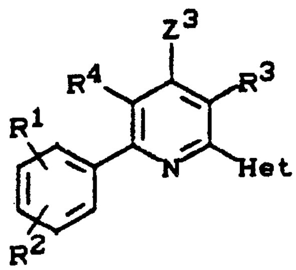

(Id)

in welcher

$R^1$, $R^2$, $R^3$, $R^4$ und Het die oben angegebene Bedeutung haben, und

$Z^3$ für Wasserstoff, jeweils gegebenenfalls substituiertes Alkyl, Alkenyl oder Alkinyl steht,

wenn man

die erfindungsgemäßen 2-Aryl-6-hetarylpyridin-Derivate der Formel (Ic)

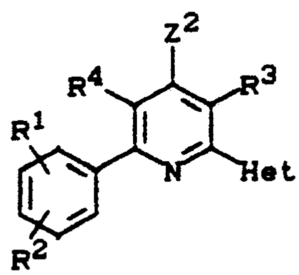

(Ic)

in welcher

$R^1$, $R^2$, $R^3$, $R^4$ und Het die oben angegebene Bedeutung haben, und

$Z^2$ für Halogen steht,

α) entweder gegebenenfalls in Gegenwart eines Verdünnungsmittels, gegebenenfalls in Gegenwart einer Base, in Gegenwart eines Katalysators und unter Wasserstoffdruck enthalogeniert, oder

β) mit Diester-Derivaten der Formel (VII)

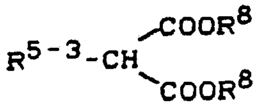

(VII)

in welcher

$R^{5-3}$ für Wasserstoff oder jeweils gegebenenfalls substituiertes Alkyl, Alkenyl oder Alkinyl steht, und

$R^8$ für Methyl oder Ethyl steht,

gegebenenfalls in Gegenwart eines Verdünnungsmittels und gegebenenfalls in Gegenwart einer Base umsetzt und die so erhaltenen Verbindungen gegebenenfalls in Gegenwart eines Verdünnungsmittels und in Gegenwart einer Base hydrolysiert und anschließend in Gegenwart einer Säure decarboxyliert.

Schließlich wurde gefunden, daß die neuen 2-Aryl-6-hetarylpyridin-Derivate der Formel (I) eine sehr gute herbizide Wirksamkeit besitzen.

Die Reste ($R^1$, $R^2$, Het usw.) die in den Wirkstoffen der Formel (I) definiert sind, haben in den Zwischen- und Vorprodukten für alle Definitionsbereiche ebenfalls die bei den Verbindungen der Formel (I) angegebenen Bedeutungen. Entsprechendes gilt auch für die Reste, die in Vor- und Zwischenprodukten mehrfach genannt sind.

Für den Begriff Alkyl in den Definitionen von $R^1$, $R^3$, $R^4$, $R^5$ und $R^6$ steht geradkettiges oder verzweigtes Alkyl mit vorzugsweise 1 bis 6, besonders bevorzugt 1 bis 4 und insbesondere 1 oder 2 Kohlenstoffatomen. Beispielhaft seien Methyl, Ethyl, n-Propyl, i-Propyl, n-Butyl, sec-Butyl, i-Butyl, t-Butyl, n-Pentyl, i-Pentyl und t-Pentyl genannt.

Für den Begriff Alkoxy in der Definition von $R^1$ steht geradkettiges oder verzweigtes Alkoxy mit vorzugsweise 1 bis 6, besonders bevorzugt 1 bis 4 Kohlenstoffatomen je Alkylrest; beispielhaft und vorzugsweise seien genannt: Methoxy, Ethoxy, n- und i-Propoxy, n-, i-, s-und t-Butoxy, n-Hexoxy und i-Hexoxy.

Für den Begriff Halogenalkyl steht in den Definitionen von $R^1$ und $R^2$ geradkettiges oder verzweigtes Alkyl mit vorzugsweise 1 bis 6 Kohlenstoffatomen, besonders bevorzugt mit 1 bis 4 Kohlenstoffatomen und 1 bis 13, vorzugsweise 1 bis 6, gleichen oder verschiedenen Halogenatomen, beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluormethyl, Trifluorchlorethyl, Chlorbutyl, Fluorbutyl.

Der Begriff Halogen steht in den Definitionen von $R^1$, $R^2$ und Z im allgemeinen für Fluor, Chlor, Brom und Iod, bevorzugt für Fluor, Chlor und Brom und besonders bevorzugt für Fluor und Chlor.

Für die Begriffe Alkenyl und Alkinyl stehen in den Definitionen von $R^{5-1}$ und $R^{5-2}$ im allgemeinen geradkettiges oder verzweigtes Alkenyl oder Alkinyl mit vorzugsweise 2 bis 6, besonders bevorzugt 2 bis 4 und ganz besonders bevorzugt 2 oder 3 Kohlenstoffatomen. Beispielhaft seien Vinyl, Allyl, Propenyl-(2)-, 1-Butenyl, 2-Butenyl, 3-Butenyl, Ethinyl, 1-Propinyl, 2-Propinyl, 1-Butinyl, 2-Butinyl, 3-Butinyl genannt.

Für den Fall, daß in der Definition von Z die beiden Reste $R^{5-2}$ und $R^6$ gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten, und durch 1 oder 2 Alkylgruppen vorzugsweise durch eine Alkylgruppe substituiert sein. Beispielhaft seien genannt: Pyrrolidino, Piperidino, Morpholino, N-Methylpiperazino, Thiomorpholino und 2,6-Dimethylmorpholino.

Die Substituenten für die in Hat aufgezählten Heterocyclen oder in Begriffen wie gegebenenfalls substituiertes Alkyl, gegebenenfalls substituiertes Alkenyl oder gegebenenfalls substituiertes Alkinyl in der Definition von Z haben die im folgenden angegebenen Bedeutungen.

Halogen als Substituent steht im allgemeinen für Fluor, Chlor, Brom und Iod, bevorzugt für Fluor, Chlor und Brom, besonders bevorzugt für Fluor und Chlor.

Alkyl als Substituent steht im allgemeinen für geradkettiges oder verzweigtes Alkyl, bevorzugt mit 1 bis 6, besonders bevorzugt mit 1 bis 4 Kohlenstoffatomen, ganz besonders bevorzugt sind Methyl und Ethyl.

Alkoxy als Substituent steht im allgemeinen für geradkettiges oder verzweigtes Alkoxy mit 1 bis 6, besonders bevorzugt 1 bis 4 Kohlenstoffatomen im jeweiligen Alkylrest; beispielhaft seien genannt: Methoxy, Ethoxy, n- und i-Propoxy, n-, i-, s- und t-Butoxy, n-Hexoxy und i-Hexoxy.

Halogenalkyl und Halogenalkoxy als Substituenten stehen im allgemeinen für geradkettiges oder verzweigtes Halogenalkyl oder Halogenalkoxy mit je 1 bis 6 Kohlenstoffatomen, besonders bevorzugt mit 1 bis 4 Kohlenstoffatomen und jeweils 1 bis 13, vorzugsweise 1 bis 9, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl, Fluorbutyl, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy,

Trichlormethoxy, Difluorethoxy, Trifluorethoxy, Tetrafluorethoxy, Trichlorethoxy, Chlordifluormethoxy und Trifluorchlorethoxy.

Alkylthio als Substituent steht im allgemeinen in den Resten für geradkettiges oder verzweigtes Alkylthio mit vorzugsweise 1 bis 6 Kohlenstoffatomen, beispielsweise sind darunter die folgenden Gruppen zu verstehen: Methylthio-, Ethylthio-, Propylthio-, Butylthio-, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Alkoxycarbonylalkyl als Substituent steht im allgemeinen für geradkettiges oder verzweigtes Alkoxycarbonylalkyl mit 1 bis 6, vorzugsweise 1 bis 4 Kohlenstoffatomen im Alkoxyrest und 1 bis 4, vorzugsweise 1 bis 2 Kohlenstoffatomen im Alkylrest; beispielhaft seien genannt: Methoxycarbonylmethyl, Ethoxycarbonylmethyl, n-Propoxycarbonylmethyl, i-Propoxycarbonylmethyl, n-, i-, s- und t-Butoxycarbonylmethyl.

Halogenalkylthio als Substituent steht im allgemeinen in den Resten für geradkettiges oder verzweigtes Halogenalkylthio mit vorzugsweise 1 bis 6 Kohlenstoffatomen, besonders bevorzugt mit 1 bis 4 Kohlenstoffatomen und jeweils 1 bis 13, vorzugsweise 1 bis 9, gleichen oder verschiedenen Halogenatomen wie unter Halogen definiert; beispielhaft seien genannt: Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthic, Tetrafluorethylthio, Trichlorethylthio, Chlordifluormethylthio und Trifluorchlorethylthio.

(Di)alkylamino steht im allgemeinen als Substituent in den Resten für eine Aminogruppe mit 1 oder 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien. Beispielhaft seien Methylamino, Ethylamino, n-Propylamino, i-Propylamino, Dimethylamino, Diethylamino, Di-n-propylamino und Di-i-propylamino aufgeführt.

Bevorzugt sind Verbindungen der Formel (I), bei welchen

$R^1$ für Wasserstoff, Halogen, jeweils geradkettiges oder verzweigtes Alkyl oder Alkoxy mit jeweils 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen steht,

$R^2$ für Wasserstoff, Halogen, geradkettiges oder verzweigtes Halogenalkyl mit 1 bis 6 Kohlenstoffatomen und 1 bis 13 gleichen oder verschiedenen Halogenatomen steht,

$R^3$ und $R^4$ unabhängig voneinander jeweils für Wasserstoff oder geradkettiges oder verzweigtes Alkyl mit 1 bis 6 Kohlenstoffatomen stehen,

Het für jeweils unsubstituiertes oder einfach oder mehrfach, gleich oder verschieden substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht, wobei als Substituenten ausgewählt sind:

Halogen, Cyano, Nitro, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Alkoxy oder Alkylthio mit jeweils 1 bis 6 Kohlenstoffatomen, jeweils geradkettiges oder verzweigtes Halogenalkylthio oder Halogenalkoxy mit jeweils 1 bis 6 Kohlenstoffatomen und jeweils 1 bis 13 gleichen oder verschiedenen Halogenatomen, Phenylamino, (Di)alkylamino mit 1 bis 6 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist,

Z für Wasserstoff, Halogen oder die Gruppierungen $-X_n-R^{5-1}$ oder

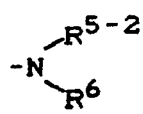

steht, wobei

X für Sauerstoff oder Schwefel steht,

$R^{5-1}$ und $R^{5-2}$ unabhängig voneinander für jeweils gegebenenfalls einfach oder mehrfach, gleich oder verschieden substituiertes, geradkettiges oder verzweigtes Alkyl mit 1 bis 8 Kohlenstoffatomen, Alkenyl mit 2 bis 6 Kohlenstoffatomen, Alkinyl mit 2 bis 6 Kohlenstoffatomen steht, wobei als Substituenten jeweils ausgewählt sind: Halogen, Cyano, geradkettiges oder verzweigtes Alkoxy mit 1 bis 6 Kohlenstoffatomen sowie geradkettiges oder verzweigtes Alkoxycarbonylalkyl mit 1 bis 6 Kohlenstoffatomen im Alkoxyteil und 1 bis 4 Kohlenstoffatomen im Alkylteil,

$R^6$ für Wasserstoff, geradkettiges oder verzweigtes Alkyl mit 1 bis 6 Kohlenstoffatomen steht, oder

$R^{5-2}$ und $R^6$ gemeinsam mit dem Stickstoffatom an, welches sie gebunden sind, einen 5- oder 6-gliedrigen gesättigten Ring bilden, welcher gegebenenfalls ein Sauerstoff-, Schwefel- oder Stickstoffatom enthält und welcher gegebenenfalls einfach oder zweifach durch Methyl und/oder Ethyl substituiert ist,

n für 0 oder 1 steht,

ausgenommen die Verbindungen 5-Methyl-3-trifluormethyl-1-(6-phenyl-4-trifluormethyl-2-pyridyl)-pyrazol, 2-(4-Dimethylamino-phenyl)-6-(2,5-dimethyl-pyrrol-1-yl)-pyridin, 6-Phenyl-(2,2'-bipyridyl), 4-Methylthio-6-phenyl-2,2'-bipyridin, 4-Methyl-2-phenyl-6-(2-thienyl)-pyridin, 6-[4-(Brommethyl)phenyl)-2,2'-bipyridin; 6-(4-Methylphenyl)-2,2'-bipyridin und 4-(Methylthio)-2-phenyl-6-(2-thienyl)-pyridin.

Besonders bevorzugt sind Verbindungen der Formel (I), bei welchen

$R^1$ für Wasserstoff, Fluor, Chlor, Brom, Methyl, Ethyl, n-Propyl, i-Propyl, n-Butyl- i-Butyl, s-Butyl, t-Butyl, Methoxy, Ethoxy, n-Propoxy, i-Propoxy, n-Butoxy, i-Butoxy, s-Butoxy, t-Butoxy oder für geradkettiges oder verzweigtes Halogenalkyl mit 1 bis 4 Kohlenstoffatomen und 1 bis 9 Fluor-und/oder Chloratomen steht,

$R^2$ für Wasserstoff, Fluor, Chlor, Brom, geradkettiges oder verzweigtes Halogenalkyl mit 1 bis 4 Kohlenstoffatomen und 1 bis 9 Fluor- und/oder Chloratomen steht,

$R^3$ und $R^4$ unabhängig voneinander jeweils für Wasserstoff, Methyl, Ethyl, n-Propyl, i-Propyl, n-Butyl, i-Butyl, s-Butyl oder t-Butyl stehen,

Het für jeweils unsubstituiertes oder 1- bis 3-fach, gleich oder verschieden substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht, wobei als Substituenten jeweils ausgewählt sind: Fluor, Chlor, Brom, Iod, Cyano, Nitro, Methyl, Ethyl, n- oder i-Propyl, n-, i-, s- oder t-Butyl, jeweils geradkettiges oder verzweigtes Halogenalkyl, Halogenalkoxy oder Halogenalkylthio mit jeweils 1 bis 4 Kohlenstoffatomen und 1 bis 9 Fluor- und/oder Chloratomen, Phenylamino oder (Di)alkylamino mit 1 bis 4 Kohlenstoffatomen in den jeweiligen geradkettigen oder verzweigten Alkylteilen, wobei im Falle von Dialkylamino die beiden Alkylteile, gemeinsam mit dem Stickstoffatom, an welches sie gebunden sind, einen 5- oder 6-gliedrigen, gesättigten Ring bilden können, welcher gegebenenfalls ein Sauerstoff-, Schwefel- oder Stickstoffatom enthält und welcher gegebenenfalls durch 1 oder 2 Methylgruppen substituiert ist,

Z für Wasserstoff, Fluor, Chlor, Brom oder die Gruppierungen $-X_n-R^{5-1}$ oder

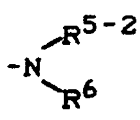

steht, wobei

X für Sauerstoff oder Schwefel steht,

$R^{5-1}$ und $R^{5-2}$ unabhängig voneinander für jeweils gegebenenfalls 1- oder 2-fach, gleich oder verschieden substituiertes Alkyl mit 1 bis 6 Kohlenstoffatomen, geradkettiges oder verzweigtes Alkenyl mit 2 bis 4 Kohlenstoffatomen, geradkettiges oder verzweigtes Alkinyl mit 2 bis 4 Kohlenstoffatomen steht, wobei als Substituenten jeweils ausgewählt sind: Fluor, Chlor, Brom, Cyano, geradkettiges oder verzweigtes Alkoxycarbonylalkyl mit 1 bis 4 Kohlenstoffatomen im Alkoxyteil und 1 oder 2 Kohlenstoffatomen im Alkylteil,

$R^6$ für Wasserstoff, Methyl, Ethyl, n- oder i-Propyl, n-, i-, s- oder t-Butyl steht, oder

$R^{5-2}$ und $R^6$ gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylguppen substituiert ist, wie insbesondere Pyrrolidino, Piperidino, Morpholino, Thiomorpholino, N-Methyl-piperazino oder 2,6-Dimethylmorpholino,

n für 0 oder 1 steht,

ausgenommen die Verbindungen 5-Methyl-3-trifluormethyl-1-(6-phenyl-4-trifluormethyl-2-pyridyl)-pyrazol 2-(4-Dimethylamino-phenyl)-6-(2,5-dimethyl-pyrrol-1-yl)-pyridin, 6-Phenyl-(2,2'-bipyridyl); 4-Methylthio-6-phenyl-2,2'-bipyridin, 4-Methyl-2-phenyl-6-(2-thienyl)-pyridin, 6-[4-(Brommethyl)phenyl]-2,2'-bipyridin, 6-(4-Methylphenyl)-2,2'-bipyridin, 4-(Methylthio)-2-phenyl-6-(2-thienyl)-pyridin.

Ganz besonders bevorzugt sind Verbindungen der Formel (I), bei welchen

$R^1$ für Wasserstoff, Fluor, Chlor, Brom, Methyl, Ethyl, n-Propyl, i-Propyl, Methoxy, Ethoxy, Trifluormethyl, Difluormethyl oder Trichlormethyl steht,

$R^2$ für Wasserstoff, Fluor, Chlor, Brom, Trifluormethyl, Difluormethyl oder Trichlormethyl steht,

$R^3$ und $R^4$ unabhängig voneinander für Wasserstoff, Methyl oder Ethyl stehen,

Het für jeweils unsubstituiertes oder 1- oder 2-fach, gleich oder verschieden substituiertes Pyridyl, Pyrrolyl, Thienyl, Furyl oder Thiazolyl steht, wobei als Substituenten ausgewählt sind: Fluor, Chlor, Brom, Methyl, Ethyl, Trifluormethyl, Methoxy sowie Methylthio,

Z für Wasserstoff, Fluor, Chlor, Brom oder die Gruppierungen $-X_n-R^{5-1}$ oder

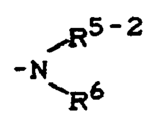

steht, wobei

X für Sauerstoff oder Schwefel steht,

$R^{5-1}$ und $R^{5-2}$ unabhängig voneinander für Methyl, Ethyl, n-Propyl, i-Propyl, n-Butyl, s-Butyl, i-Butyl, t-Butyl, Methoxymethyl, Ethoxymethyl, Methoxycarbonylmethyl, Ethoxycarbonylmethyl, Allyl oder Propargyl steht,

$R^6$ für Wasserstoff, Methyl, Ethyl, n-Propyl oder i-Propyl steht oder

$R^{5-2}$ und $R^6$ gemeinsam mit dem Stickstoffatom, an welches sie gebunden sind, für Pyrrolidino, Piperidino, Morpholino, Thiomorpholino, N-Methyl-piperazino oder 2,6-Dimethylmorpholino stehen und

n für 0 oder 1 steht.

ausgenommen die bereits oben genannten Verbindungen.

Im einzelnen seien außer den bei den Herstellungsbeispielen genannten Verbindungen die folgenden 2-Aryl-6-hetarylpyridin-Derivate der allgemeinen Formel (I) genannt:

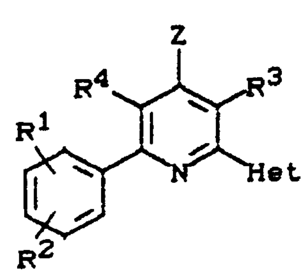

(I)

Tabelle 1

| $R^1$ | $R^2$ | $R^3$ | $R^4$ | Z | Het |
|---|---|---|---|---|---|
| 3-$CF_3$ | H | H | H | $-OCH_3$ | 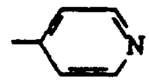 |
| 3-$CF_3$ | H | H | H | $-OC_2H_5$ | 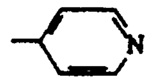 |
| 3-$CF_3$ | H | H | H | $-OCH_2-CH=CH_2$ | 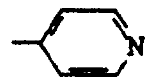 |
| 3-$CF_3$ | H | H | H | $-OCH_2-C\equiv CH$ | 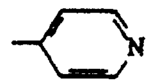 |
| 3-$CF_3$ | H | H | H | $-SCH_3$ | 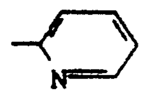 |
| 3-$CF_3$ | H | H | H | $-OC_2H_5$ | 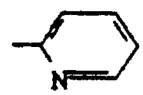 |
| 3-$CF_3$ | H | $CH_3$ | H | $-OCH_3$ | 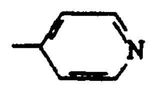 |
| 2-$CF_3$ | H | H | H | $-SCH_3$ | 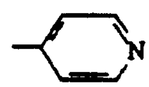 |
| 2-$CF_3$ | H | H | H | $-OCH_3$ | 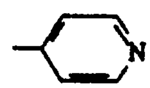 |
| 2-$CF_3$ | H | H | H | $-OC_2H_5$ | 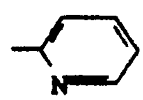 |

**Tabelle 1** (Fortsetzung)

| $R^1$ | $R^2$ | $R^3$ | $R^4$ | Z | Het |
|---|---|---|---|---|---|
| 2-$CF_3$ | H | H | H | $-OC_2H_5$ | 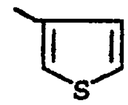 |
| 2-$CF_3$ | H | H | H | $-OCH_2-C{\equiv}CH$ | 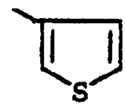 |
| 4-$CF_3$ | H | H | H | $-SCH_3$ | 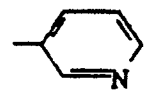 |
| 4-$CF_3$ | H | H | H | $-SC_2H_5$ | 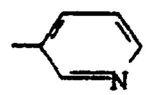 |
| 4-$CF_3$ | H | H | H | $-OC_2H_5$ | 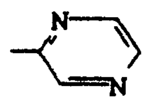 |
| 4-$CF_3$ | H | $CH_3$ | H | $-OCH_3$ | 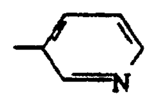 |
| 3-$CF_3$ | 5-$CF_3$ | H | H | $-OC_2H_5$ | 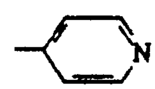 |
| 3-$CF_3$ | 5-$CF_3$ | H | H | $-OC_2H_5$ | 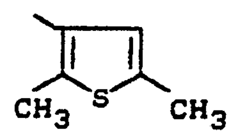 |
| 3-$CF_3$ | 5-$CF_3$ | H | H | 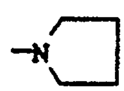 | 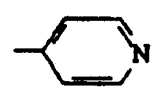 |

<u>Tabelle 1</u> (Fortsetzung)

| $R^1$ | $R^2$ | $R^3$ | $R^4$ | Z | Het |
|---|---|---|---|---|---|
| 3-Cl | H | H | H | $-SC_2H_5$ | 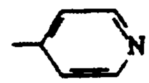 |
| 3-Cl | H | H | H | $-OCH_3$ | 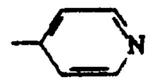 |
| 3-Cl | H | H | H | $-OC_3H_7$-n | 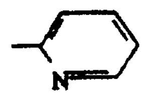 |
| 3-Cl | H | H | $CH_3$ | $-OCH_3$ | 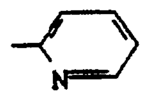 |
| 3-Cl | H | H | H | $-OC_2H_5$ | 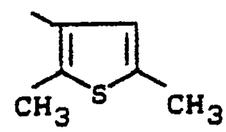 |
| 3-Cl | H | H | H | $-OCH_2-CH{=}CH_2$ | 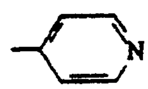 |
| 3-Cl | H | $CH_3$ | H | 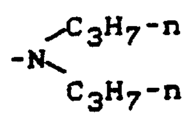 | 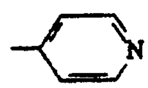 |
| 3-Cl | H | H | H | 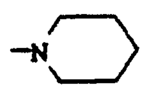 | 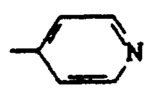 |
| 3-Cl | H | H | H | $-CH_3$ | 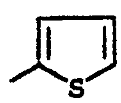 |
| 3-Cl | H | H | $CH_3$ | $-OCH_3$ | 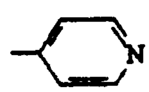 |

Tabelle 1 (Fortsetzung)

| $R^1$ | $R^2$ | $R^3$ | $R^4$ | Z | Het |
|---|---|---|---|---|---|
| 3-Cl | H | H | H | $-OC_2H_5$ | 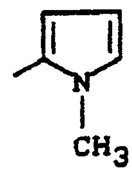 |
| 3-Cl | H | $CH_3$ | H | $-OCH_2-C{\equiv}CH$ | 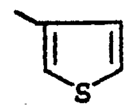 |
| 2-Cl | H | H | H | $-OC_2H_5$ | 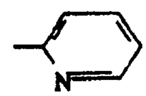 |
| 2-Cl | H | H | H | $-OC_2H_5$ | 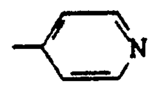 |
| 2-Cl | H | H | H | $-OC_2H_5$ | 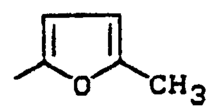 |
| 2-Cl | H | H | H | $-SC_2H_5$ | 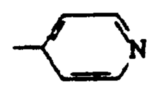 |
| 2-Cl | H | H | $CH_3$ | $-OCH_3$ | 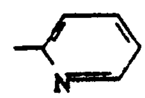 |
| 2-Cl | H | H | H | $-OCH_3$ | 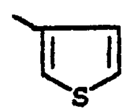 |
| 2-Cl | H | H | H | $-CH_3$ | 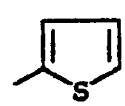 |

Tabelle 1 (Fortsetzung)

| $R^1$ | $R^2$ | $R^3$ | $R^4$ | Z | Het |
|---|---|---|---|---|---|
| 4-Cl | H | H | H | $-OCH_3$ | 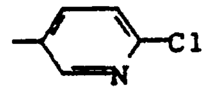 |
| 4-Cl | H | H | H | $-OC_2H_5$ | 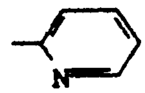 |
| 4-Cl | H | H | H | $-OCH_2-CH=CH_2$ | 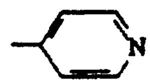 |
| 4-Cl | H | $-C_2H_5$ | H | $-OC_3H_7$-n | 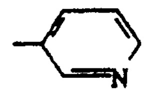 |
| 4-Cl | H | H | H | $-N(C_2H_5)_2$ | 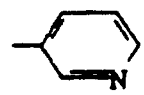 |
| 4-Cl | H | H | $CH_3$ | $-N(CH_3)_2$ | 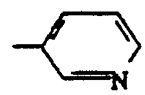 |
| 2-Cl | 4-Cl | H | H | $-SCH_3$ | 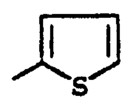 |
| 2-Cl | 4-Cl | H | H | $-SCH_3$ | 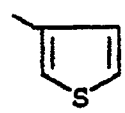 |
| 2-Cl | 4-Cl | H | H | $-SC_2H_5$ | 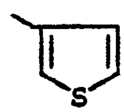 |
| 2-Cl | 4-Cl | H | H | $-OCH_3$ | 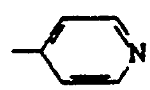 |

Tabelle 1 (Fortsetzung)

| $R^1$ | $R^2$ | $R^3$ | $R^4$ | Z | Het |
|---|---|---|---|---|---|
| 2-Cl | 4-Cl | H | H | $-OCH_3$ | 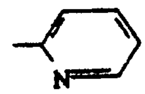 |
| 2-Cl | 4-Cl | H | H | $-OC_2H_5$ | 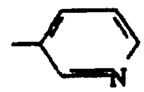 |
| 3-Cl | 5-Cl | H | H | $-OC_2H_5$ | 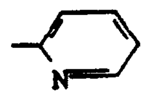 |
| 3-Cl | 5-Cl | H | H | $-OCH_2-CH=CH_2$ | 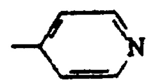 |
| 3-Cl | 5-Cl | H | H | $-OCH_2-C\equiv CH$ | 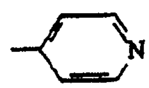 |
| 3-Cl | 5-Cl | H | H | $-CH_3$ | 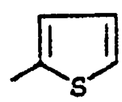 |
| 3-Cl | 5-Cl | $CH_3$ | H | 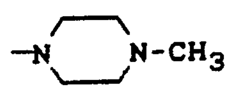 | 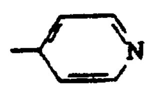 |
| 2-Cl | 3-Cl | H | H | $-SCH_3$ | 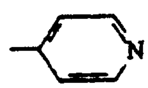 |
| 3-Cl | 4-Cl | H | H | $-SCH_3$ | 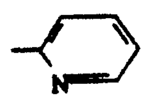 |
| 3-Cl | 4-Cl | H | $CH_3$ | $-OCH_3$ | 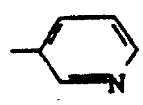 |

<u>Tabelle 1</u> (Fortsetzung)

| $R^1$ | $R^2$ | $R^3$ | $R^4$ | Z | Het |
|---|---|---|---|---|---|
| 2-Cl | 5-Cl | H | H | $-OC_2H_5$ | 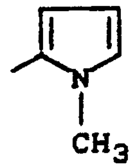 |
| 3-F | H | H | H | $-OCH_3$ | 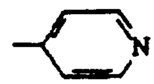 |
| 3-F | H | H | H | $-OCH_3$ | 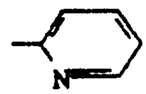 |
| 3-F | H | H | H | $-OC_2H_5$ | 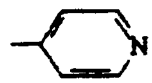 |
| 3-F | H | $CH_3$ | H | $-OC_2H_5$ | 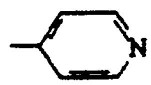 |
| 3-F | H | $-C_2H_5$ | H | $-OC_2H_5$ | 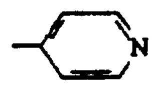 |
| 3-F | H | H | H | $-OCH_2-CH=CH_2$ | 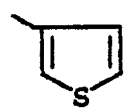 |
| 3-F | H | H | H | $-OCH_2-CH=CH_2$ | 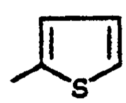 |
| 3-F | H | H | H | 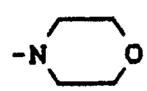 | 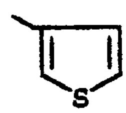 |

**Tabelle 1** (Fortsetzung)

| $R^1$ | $R^2$ | $R^3$ | $R^4$ | Z | Het |
|---|---|---|---|---|---|
| 3-F | H | H | H | 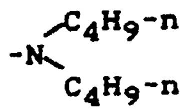 | 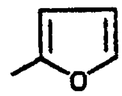 |
| 3-F | 4-$CH_3$ | H | H | 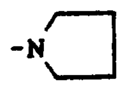 | 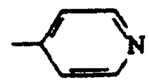 |
| 3-F | 4-$CH_3$ | H | H | $-CH_3$ | 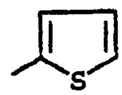 |
| 2-F | H | H | H | $-OC_2H_5$ | 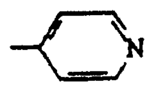 |
| 2-F | H | H | H | $-OC_2H_5$ | 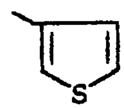 |
| 2-F | H | H | $CH_3$ | $-OC_2H_5$ | 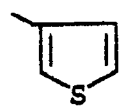 |
| 4-F | H | H | H | $-SCH_3$ | 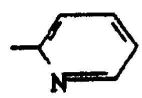 |
| 4-F | H | H | H | $-SCH_3$ | 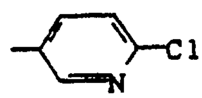 |
| 4-F | H | H | H | $-SC_2H_5$ | 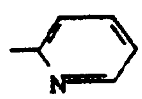 |
| 4-F | H | H | H | $-SC_2H_5$ | 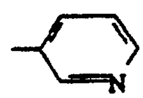 |

Tabelle 1 (Fortsetzung)

| $R^1$ | $R^2$ | $R^3$ | $R^4$ | Z | Het |
|---|---|---|---|---|---|
| 4-F | H | $CH_3$ | H | $-OCH_3$ | 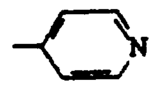 |
| 4-F | H | H | H | $-OCH_3$ | 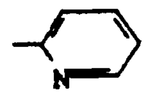 |
| 4-F | H | H | H | $-OC_2H_5$ | 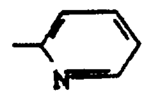 |
| 4-F | H | H | H | $-OC_2H_5$ | 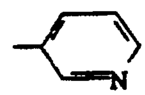 |
| 2-F | 4-F | H | H | $-SCH_3$ | 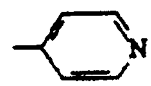 |
| 2-F | 4-F | H | H | $-OCH_3$ | 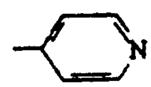 |
| 2-F | 4-F | H | H | $-SC_2H_5$ | 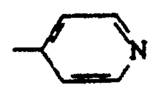 |
| 2-F | 4-F | H | H | $-OC_2H_5$ | 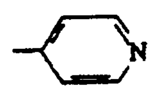 |
| 2-F | 4-F | $CH_3$ | H | $-OCH_2-CH=CH_2$ | 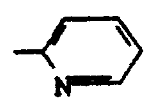 |
| 2-F | 4-F | H | H | $-OCH_2-C\equiv CH$ | 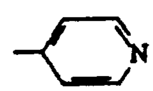 |

Tabelle 1 (Fortsetzung)

| $R^1$ | $R^2$ | $R^3$ | $R^4$ | Z | Het |
|---|---|---|---|---|---|
| 2-F | 4-F | H | H | $-OC_2H_5$ | 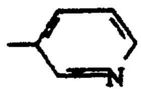 |
| 2-F | 4-F | H | H | $-OC_2H_5$ | 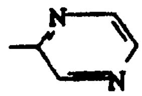 |
| 2-F | 4-F | H | H | $-SC_2H_5$ | 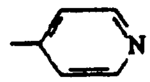 |
| 3-F | 4-F | H | H | $-OCH_3$ | 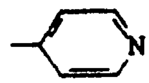 |
| 3-F | 4-F | H | H | $-OC_2H_5$ | 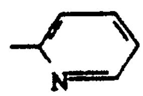 |
| 3-F | 4-F | H | H | $-OC_2H_5$ | 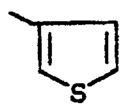 |
| $3-CH_3$ | H | H | H | $-SCH_3$ | 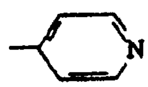 |
| $3-CH_3$ | H | H | H | $-SCH_3$ | 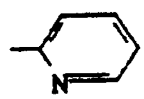 |
| $3-CH_3$ | H | H | H | $-OCH_3$ | 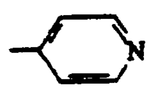 |
| $3-CH_3$ | H | $-C_2H_5$ | H | $-OCH_3$ | 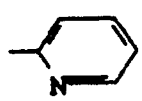 |

<u>Tabelle 1</u> (Fortsetzung)

| $R^1$ | $R^2$ | $R^3$ | $R^4$ | Z | Het |
|---|---|---|---|---|---|
| 3-$CH_3$ | H | H | H | -$OC_2H_5$ | 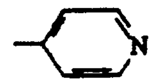 |
| 3-$CH_3$ | H | H | H | -$OC_2H_5$ | 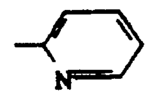 |
| 2-$CH_3$ | H | H | H | -$OC_2H_5$ | 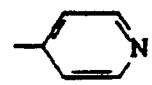 |
| 2-$CH_3$ | H | $CH_3$ | H | -$OCH_3$ | 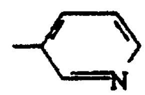 |
| 2-$CH_3$ | H | $CH_3$ | H | -$OC_2H_5$ | 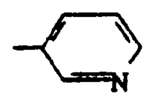 |
| 4-$CH_3$ | H | H | H | -$OCH_3$ | 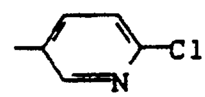 |
| 4-$CH_3$ | H | H | $CH_3$ | -$OCH_3$ | 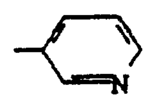 |
| 3-$C_2H_5$ | H | H | H | -$OC_2H_5$ | 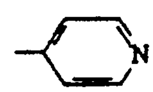 |
| 3-$C_2H_5$ | H | H | H | -$OC_2H_5$ | 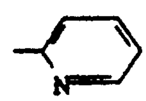 |
| 3-$C_2H_5$ | H | H | H | -$SC_2H_5$ | 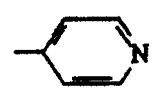 |

Tabelle 1 (Fortsetzung)

| $R^1$ | $R^2$ | $R^3$ | $R^4$ | Z | Het |
|---|---|---|---|---|---|
| $3-C_2H_5$ | H | H | H | $-OCH_2-CH=CH_2$ | 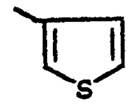 |
| $3-C_2H_5$ | H | H | H | $-OCH_2-CH=CH_2$ | 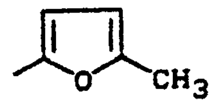 |
| $3-C_2H_5$ | H | H | H | 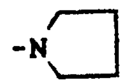 | 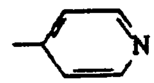 |
| $3-C_2H_5$ | H | H | H | $-CH_3$ | 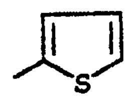 |
| $4-C_2H_5$ | H | H | H | $-OC_2H_5$ | 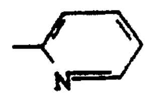 |
| $3-OCH_3$ | H | H | H | $-OC_2H_5$ | 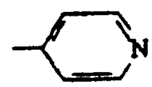 |
| $3-OCH_3$ | H | H | H | $-OC_2H_5$ | 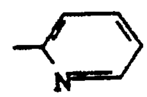 |
| $3-OCH_3$ | H | H | H | $-OC_2H_5$ | 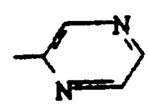 |
| $3-OCH_3$ | H | $CH_3$ | H | $-OC_2H_5$ | 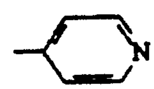 |
| $3-OCH_3$ | H | $CH_3$ | H | $-SC_2H_5$ | 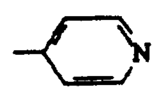 |

Tabelle 1 (Fortsetzung)

| $R^1$ | $R^2$ | $R^3$ | $R^4$ | Z | Het |
|---|---|---|---|---|---|
| $3\text{-}OCH_3$ | H | H | H | 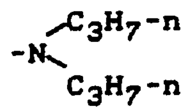 | 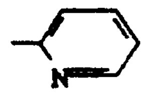 |
| $2\text{-}OCH_3$ | H | H | H | $-SCH_3$ | 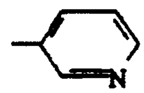 |
| $2\text{-}OCH_3$ | H | H | H | $-OC_2H_5$ | 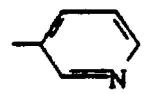 |
| $2\text{-}OCH_3$ | H | H | H | $-CH_3$ | 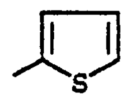 |
| H | H | H | $CH_3$ | $-OC_2H_5$ | 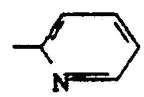 |
| H | H | $CH_3$ | H | $-OC_2H_5$ | 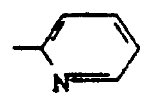 |
| H | H | $-C_2H_5$ | H | $-OCH_3$ | 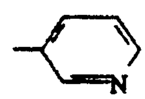 |
| H | H | H | $CH_3$ | $-OC_2H_5$ | 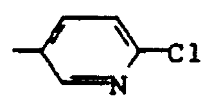 |
| H | H | H | $CH_3$ | $-OC_2H_5$ | 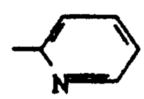 |
| H | H | H | $-C_2H_5$ | $-OC_2H_5$ | 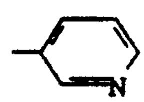 |

Verwendet man beispielsweise 3-Methylthio-1-(4-pyridyl)-5-(3-trifluorphenyl)-2-penten-1,5-dion und Ammoniumacetat als Ausgangsstoffe, so läßt sich der Reaktionsablauf des erfindungsgemäßen Verfahrens (a) durch das folgende Formelschema darstellen:

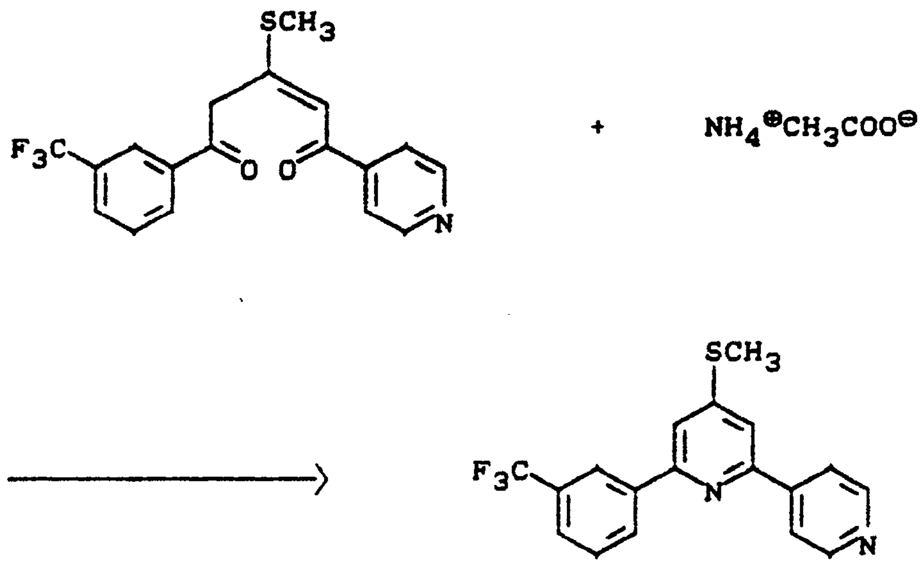


Verwendet man beispielsweise 2-(3-Chlorphenyl)-4-methylsulfonyl-6-(4-pyridyl)-pyridin und Kaliumethylat als Ausgangsstoffe, so läßt sich der Reaktionsablauf des erfindungsgemäßen Verfahrens (b-α) durch das folgende Formelschema darstellen:

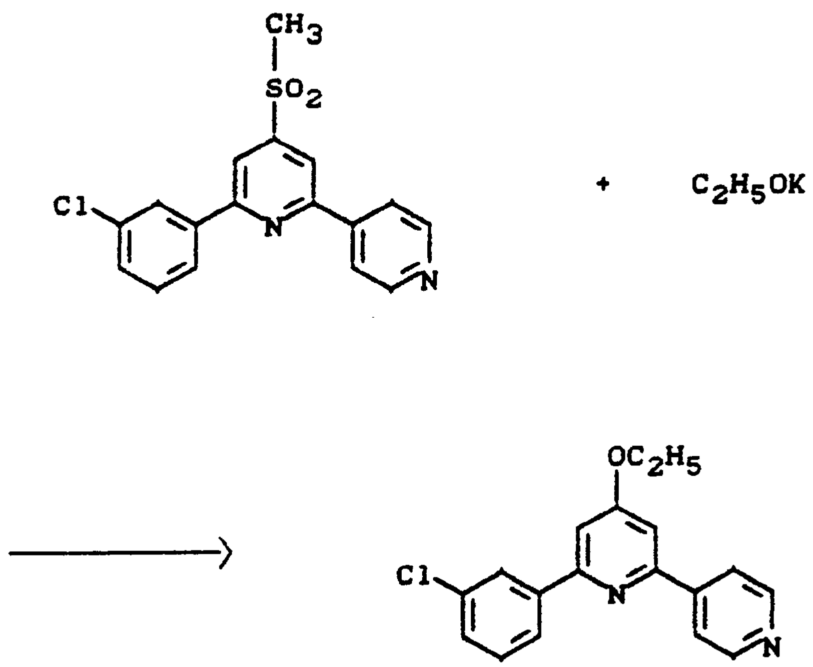


Verwendet man beispielsweise 2-(3-Chlorphenyl)-4-methylsulfonyl-6-(4-pyridyl)-pyridin und Diethylamin als Ausgangsstoffe, so läßt sich das erfindungsgemäße Verfahren (b-β) durch das folgende Formelschema darstellen:

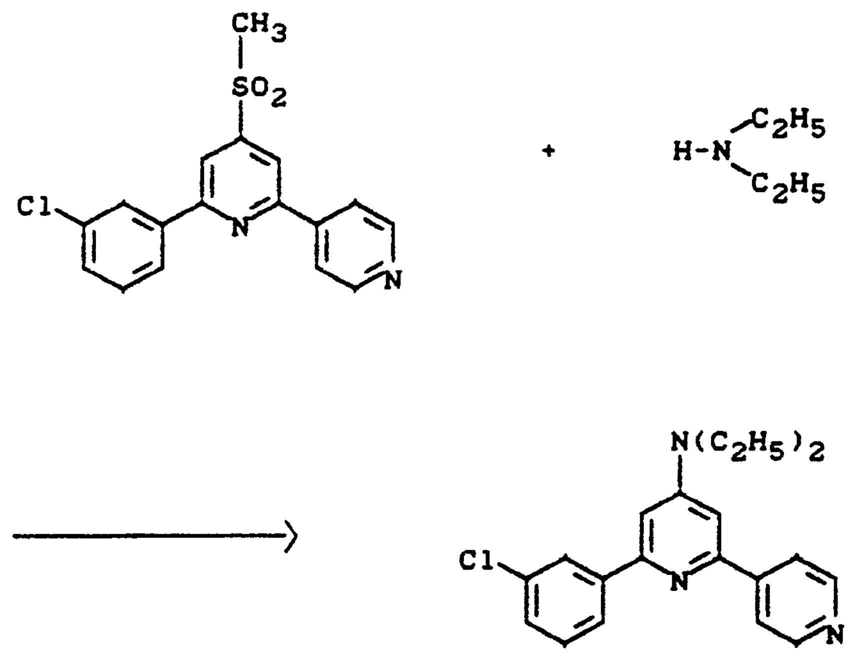

Verwendet man beispielsweise 2-(4-Chlorphenyl)-4-hydroxy-6-(3-pyridyl)-pyridin und Phosphorylchlorid als Ausgangsstoffe, so läßt sich das erfindungsgemäße Verfahren (c) durch das folgende Formelschema darstellen:

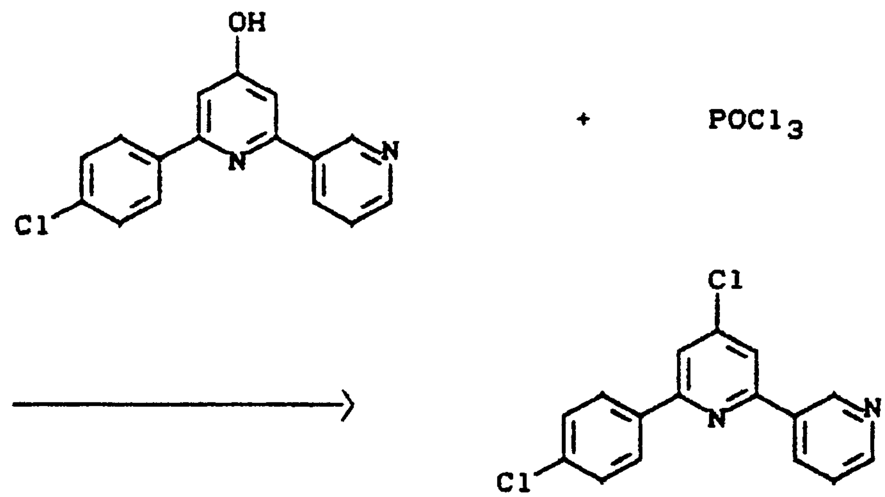

Verwendet man beispielsweise 2-(4-Chlorphenyl)-4-Chlor-6-(3-pyridyl)-pyridin als Ausgangsstoff und Wasserstoff sowie Palladium-Kohle als Katalysator, so läßt sich das erfindungsgemäße Verfahren (d-α) durch das folgende Formelschema darstellen:

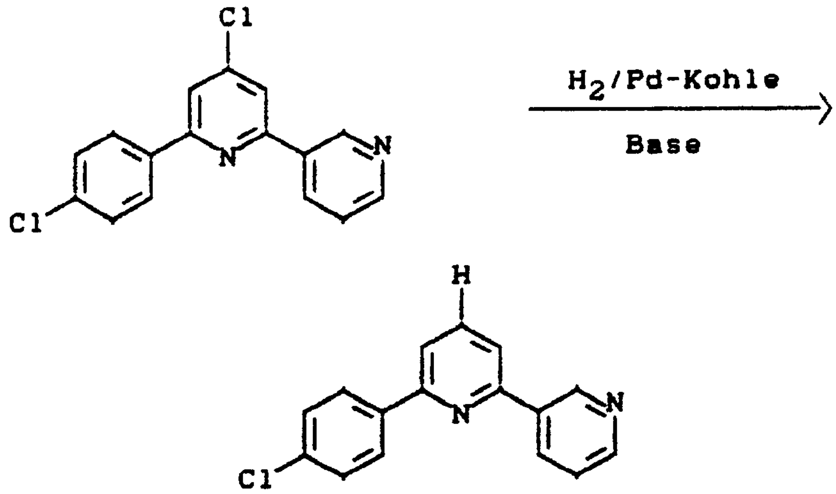


Verwendet man beispielsweise 2-(4-Chlorphenyl)-4-chlor-6-(2-thienyl)-pyridin und Malonsäurediethylester als Ausgangsstoffe, so läßt sich das erfindungsgemäße Verfahren (d-$\beta$) durch das folgende Formelschema darstellen:

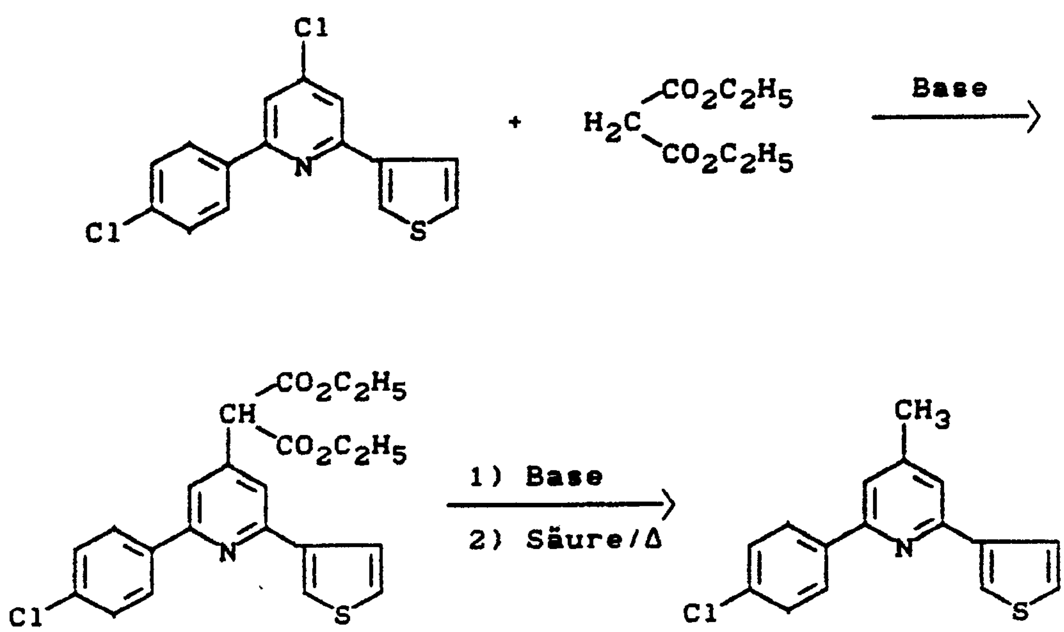


Die Verbindungen der Formel (II) und (IIa) sind teilweise bekannt. Sie lassen sich analog zu bekannten Verfahren herstellen, indem man beispielsweise entweder Ketone der Formel (VIII)

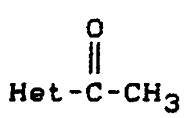

(VIII)

in welcher
Het die oben angegebene Bedeutung hat,
mit arylsubstituierten $\alpha$-Oxoketendithioacetal-Derivaten der Formel (IX)

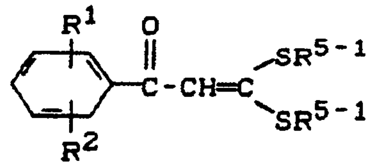

(IX)

in welcher
$R^1$, $R^2$ und $R^{5-1}$ die oben angegebene Bedeutung haben,
oder indem man Ketone der Formel (X)

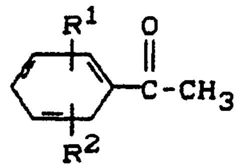

(X)

in welcher
$R^1$ und $R^2$ die oben angegebene Bedeutung haben,
mit hetarylsubstituierten α-Oxoketendithioacetal-Derivaten der Formel (XI)

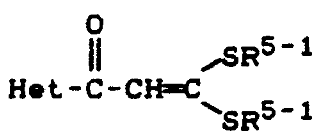

(XI)

in welcher
$R^{5-1}$ und Het die oben angegebene Bedeutung haben,
gegebenenfalls in Gegenwart eines Verdünnungsmittels wie Diethylether, Dioxan, Diethylenglykoldimethylether oder Tetrahydrofuran und gegebenenfalls in Gegenwart einer Base wie beispielsweise Natriumhydrid oder Kalium-tert-butylat bei Temperaturen zwischen 0 °C und +50 °C umsetzt (vergleiche EP 0 263 958).

Arylsubstituierte α-Oxoketendithioacetal-Derivate der Formel (IX) sowie hetarylsubstituierte α-Oxoketendithioacetal-Derivate der Formel (XI) sind ebenfalls teilweise bekannt und/oder können nach bekannten Verfahren hergestellt werden, indem man beispielsweise Acetylverbindungen der Formel (XII)

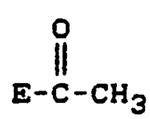

(XII)

in welcher
E für die Bedeutung von Het oder

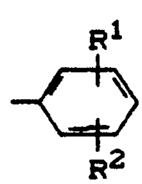

steht,
wobei
Het, $R^1$ und $R^2$ die oben angegebene Bedeutung haben,
mit Schwefelkohlenstoff, gegebenenfalls in Gegenwart eines Verdünnungsmittels wie N,N-Dimethylformamid oder Dioxan in Gegenwart einer Base wie beispielsweise Kalium-tert-butylat oder Natriumhydrid und anschließend mit einem Alkylierungsreagenz der Formel (XIII)

$R^{5-1}$-$Y^1$ (XIII)

in welcher
$R^{5-1}$ die oben angegebene Bedeutung hat, und
$Y^1$ für Chlor, Brom oder Jod steht,
bei Temperaturen zwieschen 0 °C und +50 °C umsetzt (vergleiche J. Chem. Soc., Perkin Trans. 1 (6), 549-553 (1978)).

Acetophenon-Derivate der Formel (X), Ketone der Formel (VIII), Acetylverbindungen der Formel (XII) sowie Alkylierungsreagenzien der Formel (XIII) sind allgemein bekannte Verbindungen der organischen Chemie.

In Formel (III) steht $R^7$ vorzugsweise für geradkettiges oder verzweigtes Alkyl mit 1 bis 4 Kohlenstoffatomen, insbesondere für Methyl oder Ethyl.

Die 4-Sulfonylalkyl-2-aryl-6-hetarylpyridin-Derivate der Formel (III) sind neu. Sie lassen sich jedoch analog zu bekannten Verfahren herstellen (vergleiche EP 0 263 958), indem man 2-Aryl-6-hetarylpyridin-Derivate der Formel (Ia)

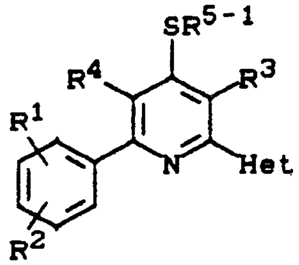

(Ia)

in welcher
$R^1$, $R^2$, $R^3$, $R^4$, $R^{5-1}$ und Het die oben angegebene Bedeutung haben,
mit einem Oxidationsmittel wie beispielsweise Metachlorperbenzoesäure oder Wasserstoffperoxid/Ameisensäure, gegebenenfalls in Gegenwart eines Katalysators wie beispielsweise Ammoniummolybdat, gegebenenfalls in Gegenwart eines Verdünnungsmittels wie beispielsweise Hexan, Heptan, Petrolether, Benzol, Toluol, Chlorbenzol, Diethylether, Dioxan, Tetrahydrofuran, Aceton, Methanol, Ethanol, Wasser oder Mischungen dieser Verdünnungsmittel, bei Temperaturen zwischen 0 °C und +130 °C umsetzt.

In Formel (IV) steht M für ein Alkalimetallkation, vorzugsweise für ein Natrium- oder Kaliumkation.

Die Verbindungungen der Formel (IV) sowie die Amine der Formel (V) sind allgemein bekannte Verbindungen der organischen Chemie.

Die 4-Hydroxy-2-aryl-6-hetarylpyridin-Derivate der Formel (VI) sind neu. Sie lassen sich jedoch analog zu bekannten Verfahren (vergleiche EP-A 0 263 958) herstellen, indem man 4-Sulfonylalkyl-2-aryl-6-hetarylpyridin-Derivate der Formel (III)

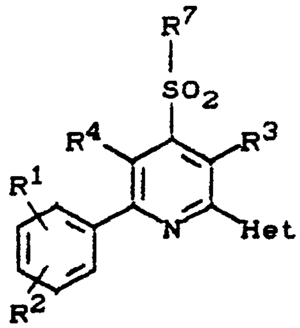

(III)

in welcher

$R^1$, $R^2$, $R^3$, $R^4$, $R^7$ und Het die oben angegebene Bedeutung haben,

mit Alkalihydroxid wie beispielsweise Natrium- oder Kaliumhydroxid, gegebenenfalls in Gegenwart eines Verdünnungsmittels wie beispielsweise Methanol, Ethanol, Wasser, Dimethylformamid, Acetonitril, Tetrahydrofuran oder Dioxan bei Temperaturen zwischen 0° C und +160° C umsetzt.

In Formel (VII) steht

$R^{5-3}$ vorzugsweise für Wasserstoff, jeweils gegebenenfalls einfach oder mehrfach, gleich oder verschieden substituiertes, geradkettiges oder verzweigtes Alkyl mit 1 bis 8 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen steht, wobei als Substituenten jeweils infrage kommen: Halogen, Cyano, geradkettiges oder verzweigtes Alkoxy mit 1 bis 6 Kohlenstoffatomen sowie geradkettiges oder verzweigtes Alkoxycarbonyl mit 1 bis 6 Kohlenstoffatomen im Alkoxyteil; besonders bevorzugt für Wasserstoff, jeweils gegebenenfalls 1- oder 2-fach, gleich oder verschieden substituiertes Alkyl mit 1 bis 6 Kohlenstoffatomen, geradkettiges oder verzweigtes Alkenyl mit 2 bis 4 Kohlenstoffatomen, geradkettiges oder verzweigtes Alkinyl mit 2 bis 4 Kohlenstoffatomen steht, wobei als Substituenten jeweils infrage kommen: Fluor, Chlor, Brom, Cyano, geradkettiges oder verzweigtes Alkoxy mit 1 bis 4 Kohlenstoffatomen sowie geradkettiges oder verzweigtes Alkoxycarbonyl mit 1 bis 4 Kohlenstoffatomen im Alkoxyteil;
Insbesondere steht $R^{5-3}$ für Wasserstoff, Methyl, Ethyl, n-Propyl, i-Propyl, n-Butyl, s-Butyl, i-Butyl, t-Butyl, Methoxymethyl, Ethoxymethyl, Methoxycarbonylmethyl, Ethoxycarbonylmethyl, Allyl oder Propargyl.

In Formel (VII) steht $R^8$ vorzugsweise für Methyl oder Ethyl.

Diester-Derivate der Formel (VII) sind allgemein bekannte Verbindungen der organischen Chemie.

Das erfindungsgemäße Verfahren (a) wird vorzugsweise unter Verwendung von Verdünnungsmitteln durchgeführt. Als Verdünnungsmittel kommen dabei praktisch alle inerten organischen Lösungsmittel infrage. Hierzu gehören Ether wie Diethylether, Diisopropylether, Dioxan, Tetrahydrofuran und Diethylenglykoldimethylether; Carbonsäuren wie Ameisensäure und Essigsäure; Alkohole wie Methanol, Ethanol, Isopropanol, tert-Butanol, Octanol, Cyclohexanol, Diethylenglykol und Glycerin; Amide wie Formamid, N,N-Dimethylsulfoxid und Sulfolan sowie Mischungen der oben genannten Verdünnungsmittel. Vorzugsweise verwendet man Tetrahydrofuran oder N,N-Dimethylformamid.

Die Reaktionstemperaturen können bei dem erfindungsgemäßen Verfahren (a) in einem größeren Bereich variiert werden. Im allgemeinen arbeitet man bei Temperaturen zwischen 0 °C und +100 °C, vorzugsweise bei Temperaturen zwischen 0 °C und +50 °C.

Zur Durchführung des erfindungsgemäßen Verfahrens (a) setzt man pro Mol an 1,5-Dioxo-Verbindung der Formel (II) im allgemeinen 1 bis 20 Mol, vorzugsweise 5 bis 10 Mol an Ammoniumsalz (beispielsweise Ammoniumacetat oder Ammoniumchlorid) oder Ammoniate ein.

Dabei ist es auch möglich, die als Ausgangsverbindungen zur Durchführung des erfindungsgemäßen Verfahrens (a) benötigten 1,5-Dioxo-Verbindungen der Formel (II) in einer vorgelagerten Reaktion direkt im Reaktionsgefäß herzustellen und direkt aus dem Reaktionsgemisch heraus ohne Isolierung weiter umzusetzen ("Eintopfverfahren").

Die Reaktionsdurchführung und Isolierung der Reaktionsprodukte erfolgt bei allen Verfahren nach allgemein üblichen Methoden.

Das erfindungsgemäße Verfahren (b-α) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Chlorbenzol und o-Dichlorbenzol, Ether wie Diethyl- und Diisopropylether, Glykoldimethylether und Diglykoldimethylether, Tetrahydrofuran und Dioxan, Nitrile wie z.B. Acetonitril und Isobutyronitril; Alkohole wie Methanol, Ethanol, Isopropanol und tert-Butanol; tertiäre Amine wie Pyridin, Triethylamin, N,N-Diethylanilin, Tributylamin und N-Methylmorpholin; Amide wie Formamid, N,N-Dimethylformamid und Acetamid; außerdem Dimethylsulfoxid und Sulfolan sowie Mischungen der oben genannten Verdünnungsmittel.

Die Reaktionstemperaturen können bei dem erfindungsgemäßen Verfahren (b-α) in einem größeren Bereich variiert werden. Im allgemeinen arbeitet man bei Temperaturen zwischen 0 °C und +150 °C, vorzugsweise bei Temperaturen zwischen 0 °C und +120 °C.

Zur Durchführung des erfindungsgemäßen Verfahrens (b-α) setzt man pro Mol an 4-Sulfonylalkyl-2-aryl-6-hetarylpyridin-Derivat der Formel (III) im allgemeinen 1 bis 15 Mol, vorzugsweise 1 bis 10 Mol an Verbindung der Formel (IV) ein.

Das erfindungsgemäße Verfahren (b-β) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon, Alkohole wie z.B. Methanol, Ethanol, Isopropanol, tert-Butanol, Carbonsäuren wie z.B. Ameisensäure und Essigsäure, Wasser sowie Dimethylsulfoxid, Tetramethylensulfon und Hexamethylphosphorsäuretriamid oder Gemische der oben genannten Verdünnungsmittel.

Die Reaktionstemperaturen können bei dem erfindungsgemäßen Verfahren (b-β) in einem größeren Bereich variiert werden. Im allgemeinen arbeitet man bei Temperaturen zwischen 0 °C und +180 °C, vorzugsweise bei Temperaturen zwischen +20 °C und +120 °C.

Zur Durchführung des erfindungsgemäßen Verfahrens (b-β) setzt man pro Mol an 4-Sulfonylalkyl-2-aryl-6-hetarylpyridin-Derivat der Formel (III) im allgemeinen 1 bis 100 Mol, vorzugsweise 1 bis 10 Mol an Amin der Formel (V) ein.

Das erfindungsgemäße Verfahren (b-β) wird vorzugsweise unter Normaldruck durchgeführt, es ist jedoch auch möglich unter erhöhtem Druck zu arbeiten.

Das erfindungsgemäße Verfahren (c) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (c) wird unter Verwendung von Halogenierungsreagenzien durchgeführt. Vorzugsweise verwendet man Thionylchlorid, Phosgen, Phosphorylchlorid, Phosphorpentachlorid, Phosphorylbromid oder Phosphortribromid.

Die Reaktionstemperaturen können bei dem erfindungsgemäßen Verfahren (c) in einem größeren Bereich variiert werden. Im allgemeinen arbeitet man bei Temperaturen zwischen 0 °C und +200 °C, vorzugsweise bei Temperaturen zwischen +50 °C und +150 °C.

Zur Durchführung des erfindungsgemäßen Verfahrens (c) setzt man pro Mol an 4-Hydroxy-2-aryl-6-hetarylpyridin-Derivat der Formel (VI) im allgemeinen 1 bis 50 Mol, vorzugsweise 1 bis 10 Mol an Halogenierungsreagenz ein.

Das erfindungsgemäße Verfahren (d-α) wird vorzugsweise unter Verwendung von Verdünnungsmitteln durchgeführt. Als Verdünnungsmittel kommen dabei praktisch alle inerten organischen Lösungsmittel infrage. Hierzu gehören Benzol, Toluol, Xylol, Tetrahydrofuran und Dioxan, Ester wie Essigsäuremethylester und -ethylester, Alkohole wie Methanol, Ethanol und Isopropanol sowie Wasser. Vorzugsweise verwendet man Wasser, Methanol, Ethanol, Essigsäureethylester und Toluol oder Mischungen dieser Verdünnungsmittel.

Die Reaktionstemperaturen können bei dem erfindungsgemäßen Verfahren (d-α) in einem größeren Bereich variiert werden. Im allgemeinen arbeitet man bei Temperaturen zwischen 0 °C und +100 °C,

vorzugsweise bei Temperaturen zwischen +20 °C und +50 °C.

Das erfindungsgemäße Verfahren (d-α) wird vorzugsweise unter Verwendung von Katalysatoren durchgeführt. Als solche kommen übliche Hydrierungskatalysatoren infrage, wie beispielsweise Raney-Nickel, elementares Platin oder Palladium, vorzugsweise verwendet man Palladium/Aktivkohle.

Das erfindungsgemäße Verfahren (d-α) wird in Gegenwart von Basen durchgeführt. Als solche kommen beispielsweise Alkalimetallhydroxide wie z.B. Natrium- und Kaliumhydroxid, Erdalkalihydroxide wie z.B. Calciumhydroxid, Alkalicarbonate, -acetate und -alkoholate, wie Natrium- und Kaliumcarbonat, Natrium- und Kaliummethylat bzw. -ethylat, Ammoniak, Amine, wie Diethylamin oder basische Ionenaustauscher infrage.

Zur Durchführung des erfindungsgemäßen Verfahrens (d-α) arbeitet man im allgemeinen bei einem Wasserstoffdruck von 1 bis 5 Atmosphären, vorzugsweise bei 1 bis 3 Atmosphären. Zur Aufarbeitung wird der Katalysator abfiltriert, die Reaktionslösung mit Wasser versetzt und mit einem organischen Lösungsmittel extrahiert. Die weitere Aufarbeitung erfolgt nach üblichen Methoden.

Zur Durchführung des erfindungsgemäßen Verfahrens (d-α) setzt man pro Mol an Verbindung der Formel (Ic) im allgemeinen 1 bis 10 Mol, vorzugsweise 1 bis 5 Mol an Wasserstoff sowie im allgemeinen 0,01 bis 1 Mol, vorzugsweise 0,01 bis 0,1 Mol an Katalysator ein.

Das erfindungsgemäße Verfahren (d-β) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Dimethylsulfoxid und Sulfolan sowie Gemische dieser Verdünnungsmittel.

Die Reaktionstemperaturen können beim ersten Reaktionsschritt des erfindungsgemäßen Verfahren (d-β) in einem größeren Bereich variiert werden. Im allgemeinen arbeitet man bei Temperaturen zwischen -10 °C und +180 °C, vorzugsweise bei Temperaturen zwischen 0 °C und +150 °C.

Der erste Reaktionsschritt des erfindungsgemäßen Verfahrens (d-β) wird in Gegenwart von Basen durchgeführt. Vorzugsweise verwendet man Alkalimetallhydride wie beispielsweise Natriumhydrid, Alkyllithium wie n-Butyllithium, Lithiumdialkylamide wie z.B. Lithiumdiisopropylamid (LDA), Alkalimetallalkoholate wie z.B. Natriummethanolat und Natriumethanolat und Metallhydroxide wie z.B. Natriumhydroxid.

Zur Durchführung des ersten Reaktionsschrittes des erfindungsgemäßen Verfahrens (d-β) setzt man pro Mol an Verbindung der Formel (Ic) im allgemeinen 0,5 bis 2 Mol, vorzugsweise 1 bis 1,5 Mol an Diester-Derivat der Formel (VII) sowie im allgemeinen 1 bis 3 Mol, vorzugsweise 1 bis 2 Mol an Base ein.

Die Reaktionstemperaturen können beim zweiten Reaktionsschritt des erfindungsgemäßen Verfahrens (d-β) in einem größeren Bereich variiert werden. Im allgemeinen arbeitet man bei Temperaturen zwischen 0 °C und +150 °C, vorzugsweise bei Temperaturen zwischen +10 °C und +100 °C.

Der zweite Reaktionsschritt des erfindungsgemäßen Verfahrens (d-β) wird in Gegenwart von Basen durchgeführt. Vorzugsweise verwendet man Alkalimetallhydroxide wie z.B. Natriumhydroxid und Alkalimetallcarbonate wie z.B. Natriumcarbonat.

Zur Durchführung des zweiten Reaktionsschrittes des erfindungsgemäßen Verfahrens (d-β) setzt man pro Mol an verwendeter Verbindung der Formel (Ic) im allgemeinen 1 bis 6 Mol, vorzugsweise 2,1 bis 5 Mol an Base ein.

Die Reaktionstemperaturen können bei der Decarboxylierungsreaktion des erfindungsgemäßen Verfahrens (d-β) in einem größeren Bereich variiert werden. Im allgemeinen arbeitet man bei Temperaturen zwischen 0 °C und +200 °C, vorzugsweise bei Temperaturen zwischen +20 °C und +150 °C.

Die Decarboxylierungsreaktion des erfindungsgemäßen Verfahrens (d-β) wird in Gegenwart von Säuren durchgeführt. Vorzugsweise verwendet man anorgansiche Säuren wie z.B. Schwefelsäure oder Chlorwasserstoffsäure und organische Säuren wie z.B. Essigsäure.

Zur Durchführung der Decarboxylierungsreaktion des erfindungsgemäßen Verfahrens (d-β) setzt man pro Mol an verwendeter Verbindung der Formel (Ic) im allgemeinen 1 bis 10 Mol, vorzugsweise 2,5 bis 6 Mol an Säure ein.

Die Durchführung des erfindungsgemäßen Verfahrens (d-β) erfolgt nach üblichen Methoden. Zur Aufarbeitung wird die Reaktionsmischung neutralisiert, konzentriert und extrahiert. Die Reinigung des Produktes erfolgt durch Chromatographie oder Umkristallisation.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei eignen sich die erfindungsgemäßen Wirkstoffe der Formel (I) besonders gut zur selektiven Bekämpfung von mono- und dikotylen Untkräutern in mono- und dikotylen Kulturen im Vor- und Nachauflaufverfahren.

Darüber hinaus besitzen einige der erfindungsgemäßen Wirkstoffe der Formel (I) fungizide Wirksamkeit z.B. gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate komman infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid(BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOPMETHYL); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOPBUTYL); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidaz ol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 2-Chlor-N-isopropylacetanilid (PROPACHLOR); 0-(6-Chlor-3-phenylpyridazin-4-yl)-S-octyl-thiocarbonat (PYRIDATE); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN) und 2-[4-(6-Chlorchinoxalin-2-yl-oxy)-phenoxy]-propionsäure-ethylester (QUIZALOFOPETHYL) infrage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

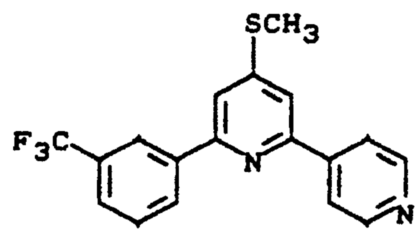

Verfahren (a)
("Eintopf-Variante")

3,8 g (0,02 Mol) 3-Trifluormethylacetophenon und 5,2 g (0,046 Mol) Kalium-tert-butylat werden in 100 ml Tetrahydrofuran 30 Minuten bei Raumtemperatur gerührt. Anschließend versetzt man die Reaktionsmischung mit 4,5 g (0,02 Mol) 3,3-Bis-(methylthio)-1-(4-pyridyl)-2-propen-1-on und rührt diese Mischung eine Stunde bei Raumtemperatur. Dann werden 100 ml konzentrierte Essigsäure und 15 g Ammoniumacetat zugesetzt und 4 Stunden am Rückfluß erhitzt, wobei das Tetrahydrofuran mittels eines Wasserabscheiders abdestilliert wird, Nach dem Abkühlen wird die Reaktionsmischung mit Natronlauge neutralisiert, mit Essigester ausgeschüttelt und über Natriumsulfat getrocknet. Die erhaltene Lösung wird unter vermindertem Druck vom Lösungsmittel befreit.

Man erhält 0,9 g (13 % der Theorie) an 4-Methylthio-6-(4-pyridyl)-2-(3-trifluormethylphenyl)-pyridin vom Schmelzpunkt 145 °C (Zersetzung).

Beispiel 2

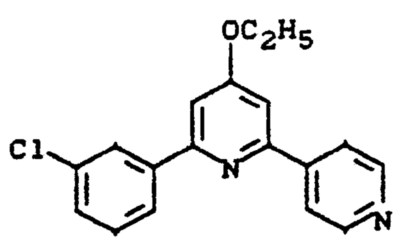

Verfahren (b-α)

1,7 g (0,005 Mol) 2-(3-Chlorphenyl)-4-methylsulfonyl-6-(4-pyridyl)-pyridin werden mit 1,3 g (0,015 Mol) Kaliumethylat in 30 ml trockenem Ethanol versetzt und die Mischung 16 Stunden am Rückfluß gekocht. Anschließend wird unter vermindertem Druck eingeengt und der Rückstand mit Methylenchlorid aufgenommen. Man wäscht die Lösung zweimal mit Wasser, trocknet über Natriumsulfat und entfernt das Lösungsmittel unter vermindertem Druck. Der Rückstand wird mit Diethylether verrührt, das erhaltene kristalline Produkt abgesaugt und getrocknet. Man erhält 0,7 g (45 % der Theorie) an 2-(3-Chlorphenyl)-4-ethoxy-6-(4-pyridyl)-pyridin vom Schmelzpunkt 123 °C.

Analog zu den Herstellungsbeispielen 1 und 2 und gemäß den allgemeinen Angaben zu den erfindungsgemäßen Verfahren (a), (b), (c) und (d) erhält man die in Tabelle 2 aufgeführten Verbindungen der allgemeinen Formel (I)

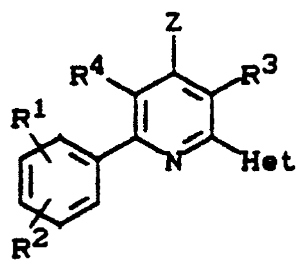

(I)

Tabelle 2

| Bsp. Nr. | $R^1$ | $R^2$ | $R^3$ | $R^4$ | Z | Het | Schmelzpunkt |
|---|---|---|---|---|---|---|---|
| 3 | 2-$CH_3$ | H | H | H | -$SCH_3$ | 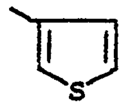 | Öl |
| 4 | 4-Cl | H | H | H | -$SCH_3$ | 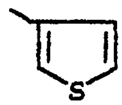 | 113° C |
| 5 | 4-Cl | H | H | H | -$SCH_3$ | 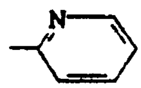 | 105° C |
| 6 | 4-Cl | H | H | H | -$SCH_3$ | 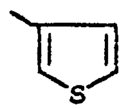 | 120° C |
| 7 | 4-Cl | H | H | H | -$SCH_3$ | 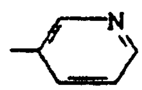 | 141° C |
| 8 | 3-Cl | H | H | H | -$SCH_3$ | 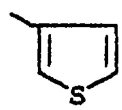 | Öl |
| 9 | 3-Cl | H | H | H | -$SCH_3$ | 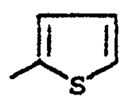 | Öl |
| 10 | 3-$CF_3$ | H | H | H | -$SCH_3$ | 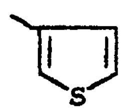 | 84° C |
| 11 | 3-$CF_3$ | H | H | H | -$SCH_3$ | 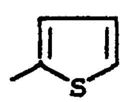 | Öl |
| 12 | 4-Cl | H | H | H | -$OC_2H_5$ | 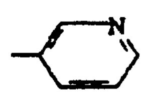 | 147° C |

<u>Tabelle 2</u> (Fortsetzung)

| Bsp. Nr. | $R^1$ | $R^2$ | $R^3$ | $R^4$ | Z | Het | Schmelzpunkt |
|---|---|---|---|---|---|---|---|
| 13 | 4-Cl | H | H | H | $-OC_2H_5$ | 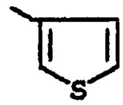 | Öl |
| 14 | 4-Cl | H | H | H | $-OC_2H_5$ | 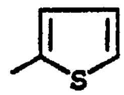 | 108° C |
| 15 | 3-$CF_3$ | H | H | H | $-SCH_3$ | 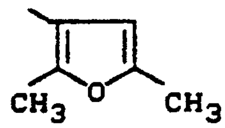 | Öl |
| 16 | 3-$CF_3$ | H | H | H | $-SCH_3$ | 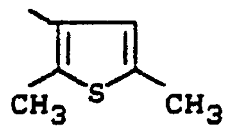 | Öl |
| 17 | 3-$CF_3$ | H | H | H | $-SCH_3$ | 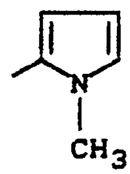 | 75° C |
| 18 | 3-$CF_3$ | H | H | H | $-SCH_3$ | 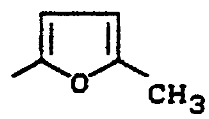 | 78° C |
| 19 | 3-$CF_3$ | H | H | H | $-SCH_3$ | 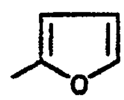 | 66° C |
| 20 | 3-$CF_3$ | H | H | H | $-SCH_3$ | 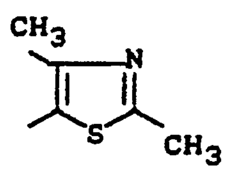 | Öl |

Tabelle 2 (Fortsetzung)

| Bsp. Nr. | $R^1$ | $R^2$ | $R^3$ | $R^4$ | Z | Het | Schmelz-punkt |
|---|---|---|---|---|---|---|---|
| 21 | 3-$CF_3$ | H | H | H | $-OC_2H_5$ | 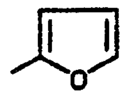 | Öl |
| 22 | 3-$CF_3$ | H | H | H | $-OC_2H_5$ | 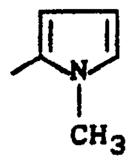 | 69° C |
| 23 | 3-$CF_3$ | H | H | H | $-OC_2H_5$ | 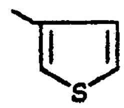 | Öl |
| 24 | 3-$CF_3$ | H | H | H | $-OC_2H_5$ | 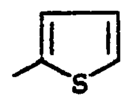 | 78° C |
| 25 | 3-$CF_3$ | H | H | H | $-OC_2H_5$ | 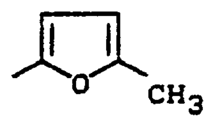 | Öl |
| 26 | 3-$CF_3$ | H | H | H | $-OC_2H_5$ | 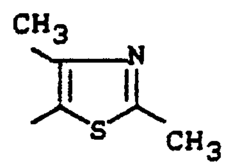 | 72° C |
| 27 | H | H | H | H | $-SCH_3$ | 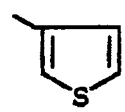 | 74° C |
| 28 | 3-$CF_3$ | H | H | H | $-OCH_3$ | 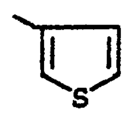 | 75° C |
| 29 | H | H | H | H | $-SCH_3$ | 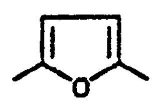 | 92° C |

**Tabelle 2** (Fortsetzung)

| Bsp. Nr. | $R^1$ | $R^2$ | $R^3$ | $R^4$ | Z | Het | Schmelz-punkt |
|---|---|---|---|---|---|---|---|
| 30 | H | H | H | H | $-SCH_3$ | 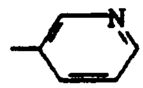 | 97° C |
| 31 | H | H | H | H | $-OC_2H_5$ | 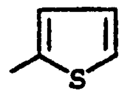 | 74° C |
| 32 | H | H | H | H | $-OC_2H_5$ | 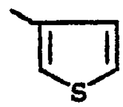 | 77° C |
| 33 | H | H | H | H | $-OC_2H_5$ | 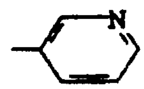 | 132-135° C |
| 34 | H | H | H | H | $-OC_2H_5$ | 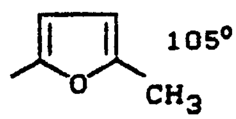 | 105° C |
| 35 | 3-Cl | H | H | H | $-SCH_3$ | 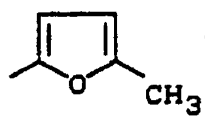 | 92° C |
| 36 | 3-Cl | H | H | H | $-OC_2H_5$ | 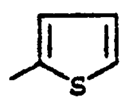 | 80° C |
| 37 | 3-Cl | H | H | H | $-OC_2H_5$ | 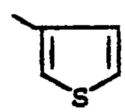 | 101° C |
| 38 | 3-Cl | H | H | H | $-OC_2H_5$ | 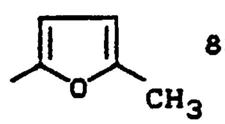 | 83° C |

Tabelle 2 (Fortsetzung)

| Bsp. Nr. | $R^1$ | $R^2$ | $R^3$ | $R^4$ | Z | Het | Schmelzpunkt |
|---|---|---|---|---|---|---|---|
| 39 | 3-Cl | H | H | H | $-SCH_3$ | 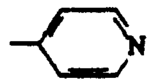 | 115° C |
| 40 | 3-Cl | H | H | H | $-OCH_3$ | 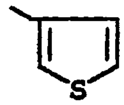 | Öl |
| 41 | 3-Cl | H | H | H | $-OC(CH_3)_3$ | 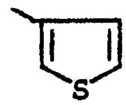 | Öl |
| 42 | 3-Cl | H | H | H | $-OC_3H_7$-n | 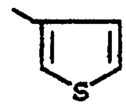 | 74° C |
| 43 | 3-Cl | 5-Cl | H | H | $-SCH_3$ | 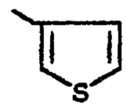 | 133° C |
| 44 | 3-Cl | H | H | $CH_3$ | $-SCH_3$ | 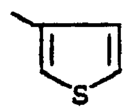 | 60° C |
| 45 | 2-F | 4-F | H | H | $-SCH_3$ | 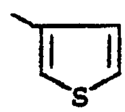 | 78° C |
| 46 | $3-CF_3$ | $5-CF_3$ | H | H | $-SCH_3$ | 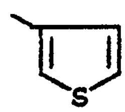 | 131° C |
| 47 | 4-Cl | H | H | $CH_3$ | $-SCH_3$ | 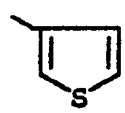 | 103° C |
| 48 | 3-Cl | H | H | H | $-OCH_2-CH=CH_2$ | 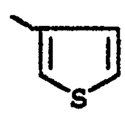 | 72° C |

<u>Tabelle 2</u> (Fortsetzung)

| Bsp. Nr. | $R^1$ | $R^2$ | $R^3$ | $R^4$ | Z | Het | Schmelzpunkt |
|---|---|---|---|---|---|---|---|
| 49 | 4-Cl | H | H | $CH_3$ | $-OC_2H_5$ | 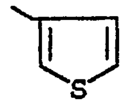 | Öl |
| 50 | 2-F | 4-F | H | H | $-OC_2H_5$ | 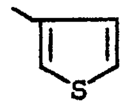 | 61° C |
| 51 | 3-Cl | 5-Cl | H | H | $-OC_2H_5$ | 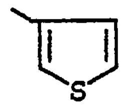 | 123° C |
| 52 | H | H | H | $CH_3$ | $-SCH_3$ | 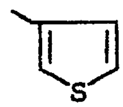 | Öl |
| 53 | 3-Cl | H | H | $CH_3$ | $-OC_2H_5$ | 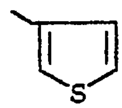 | Öl |
| 54 | H | H | H | $-C_2H_5$ | $-SCH_3$ | 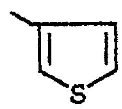 | Öl |
| 55 | H | H | H | $CH_3$ | $-OC_2H_5$ | 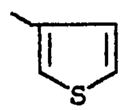 | Öl |
| 56 | 4-Cl | H | H | H | $-SCH_3$ | 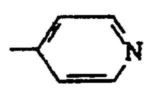 | 127° C |
| 57 | 2-Cl | H | H | H | $-SCH_3$ | 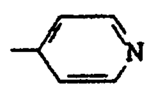 | 134° C |
| 58 | $2-CH_3$ | H | H | H | $-SCH_3$ | 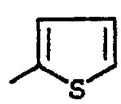 | Öl |
| 59 | 4-Cl | H | H | H | $-SCH_3$ | 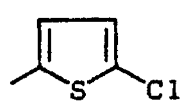 | 113-114,5° C |

Tabelle 2 (Fortsetzung)

| Bsp. Nr. | $R^1$ | $R^2$ | $R^3$ | $R^4$ | Z | Het | Schmelzpunkt |
|---|---|---|---|---|---|---|---|
| 60 | 4-Cl | H | H | H | $-SCH_3$ | 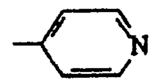 | 131-133° C |
| 61 | 4-Cl | H | H | H | $-SCH_3$ | 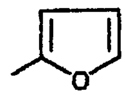 | 93-94,5° C |
| 62 | 4-Cl | H | H | H | $-SCH_3$ | 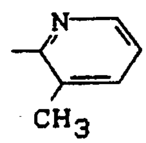 | 100-103° C |
| 63 | 4-Cl | H | H | H | $-SCH_3$ | 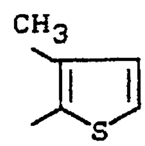 | 125-126,5° C |
| 64 | 4-Cl | H | H | H | $-SCH_3$ | 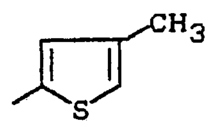 | 142-143° C |
| 65 | 4-Cl | H | H | H | $-SCH_3$ | 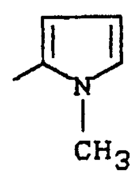 | 142-144° C |
| 66 | 4-Cl | H | H | H | $-SCH_3$ | 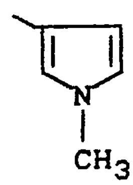 | 88-90° C |
| 67 | 4-Cl | H | H | H | $-SCH_3$ | 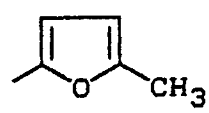 | 123-125° C |

Tabelle 2 (Fortsetzung)

| Bsp. Nr. | $R^1$ | $R^2$ | $R^3$ | $R^4$ | Z | Het | Schmelzpunkt |
|---|---|---|---|---|---|---|---|
| 68 | 4-Cl | H | H | H | $-SCH_2COOC_2H_5$ | 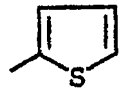 | Öl |
| 69 | 4-Cl | H | H | H | $-SCH_3$ | 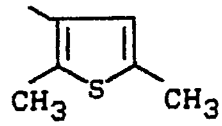 | 250-252° C |
| 70 | 4-Cl | H | H | H | $-SCH_3$ | 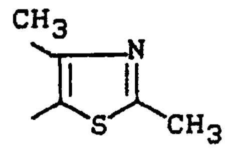 | 126-127° C |
| 71 | $2-CH_3$ | H | H | H | $-SCH_3$ | 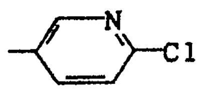 | Öl |
| 72 | $2-CH_3$ | H | H | H | $-SCH_3$ | 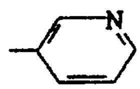 | Öl |
| 73 | $2-CH_3$ | H | H | H | $-SCH_3$ | 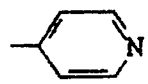 | Öl |
| 74 | 4-Cl | H | H | H | $-SCH_3$ | 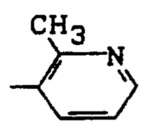 | 122-123° C |
| 75 | $2-CH_3$ | H | H | H | $-SCH_3$ | 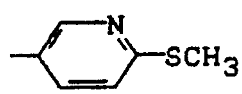 | Öl |
| 76 | $3-CF_3$ | H | H | H | $-SCH_3$ | 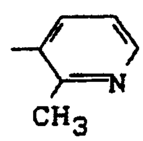 | |
| 77 | 4-F | H | H | H | $-SCH_3$ | 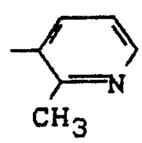 | |

Tabelle 2 (Fortsetzung)

| Bsp. Nr. | $R^1$ | $R^2$ | $R^3$ | $R^4$ | Z | Het | Schmelzpunkt |
|---|---|---|---|---|---|---|---|
| 78 | 3-$CF_3$ | H | H | H | -$SCH_3$ | 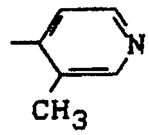 | |
| 79 | 4-F | H | H | H | -$SCH_3$ | 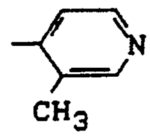 | |
| 80 | 3-Cl | H | H | H | -$SCH_3$ | 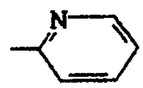 | 81° C |
| 81 | 3-Cl | H | H | H | -$SCH_3$ | 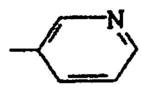 | 88° C |
| 82 | 4-Cl | H | H | H | -$SCH_3$ | 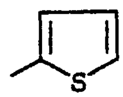 | 118-119° C |
| 83 | 4-Cl | H | H | H | -$SCH_3$ | 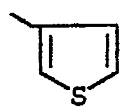 | 112-113° C |
| 84 | 4-Cl | H | H | H | -$SCH_3$ | 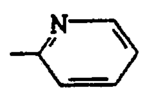 | 108-111° C |
| 85 | 4-Cl | H | H | H | -$SCH_3$ | 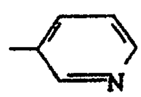 | 139,5-141° C |
| 86 | 4-Cl | H | H | H | -$OC_2H_5$ | 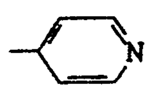 | 125° C |
| 87 | 3-Br | H | H | H | -$SCH_3$ | 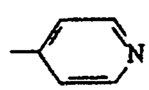 | 130° C |

Tabelle 2 (Fortsetzung)

| Bsp. Nr. | $R^1$ | $R^2$ | $R^3$ | $R^4$ | Z | Het | Schmelz-punkt |
|---|---|---|---|---|---|---|---|
| 88 | 3-Cl | H | H | H | $-OCH_3$ | 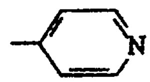 | 118° C |
| 89 | 2-Cl | H | H | H | $-SCH_3$ | 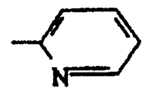 | 118° C |
| 90 | $3-CF_3$ | H | H | H | $-SCH_3$ | 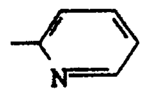 | 131° C |
| 91 | $3-CF_3$ | H | H | H | $-SCH_3$ | 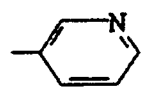 | 106° C |
| 92 | 2-Cl | H | H | H | $-SCH_3$ | 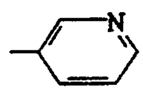 | 144° C |
| 93 | 3-Cl | H | H | H | $-SC_2H_5$ | 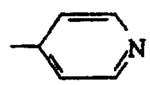 | 115° C |
| 94 | 3-Cl | H | H | H | $-SCH_3$ | 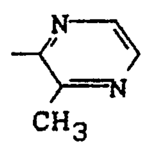 | 98° C |
| 95 | 3-Cl | H | H | H | $-OC_2H_5$ | 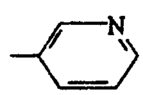 | 105° C |
| 96 | 3-Cl | H | H | H | $-OC_2H_5$ | 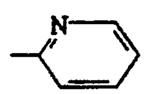 | Öl |
| 97 | 3-Cl | H | H | H | $-SC_2H_5$ | 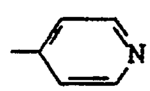 | 112° C |
| 98 | 3-Cl | H | H | H | $-OCH_3$ | 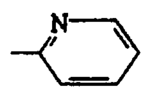 | 63° C |

Tabelle 2 (Fortsetzung)

| Bsp. Nr. | $R^1$ | $R^2$ | $R^3$ | $R^4$ | Z | Het | Schmelz-punkt |
|---|---|---|---|---|---|---|---|
| 99 | 3-Br | H | H | H | $-OCH_3$ | 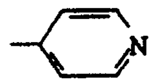 | 130° C |
| 100 | 3-Cl | H | $-CH_3$ | H | $-SCH_3$ | 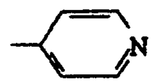 | 140° C |
| 101 | 3-Cl | H | H | H | $-OCH_2-CH=CH_2$ | 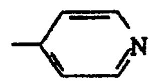 | 128° C |
| 102 | 2-Cl | H | H | H | $-OC_2H_5$ | 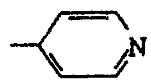 | 121° C |
| 103 | 2-Cl | H | H | H | $-OC_2H_5$ | 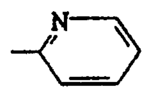 | 73° C |
| 104 | $2-CF_3$ | H | H | H | $-SCH_3$ | 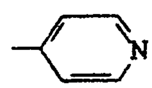 | Öl |
| 105 | 3-F | H | H | H | $-SCH_3$ | 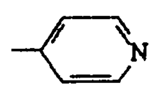 | 104° C |
| 106 | $2-CF_3$ | H | H | H | $-OC_2H_5$ | 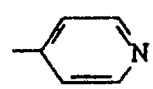 | 128° C |
| 107 | 3-F | H | H | H | $-OC_2H_5$ | 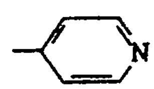 | 100° C |
| 108 | 4-Cl | H | H | H | 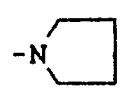 | 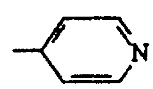 | 192° C |
| 109 | 3-Cl | H | H | H | $-NHC_3H_7n$ | 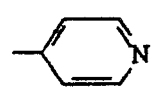 | 142° C |

Herstellung der Ausgangsverbindungen

Beispiel (III-1)

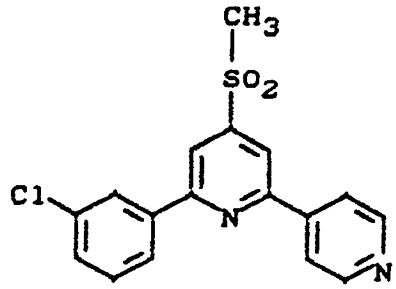

2,5 g (0,008 Mol) 4-Methylthio-2-(3-Chlorphenyl)-6-(4-pyridyl)-pyridin werden in 30 ml trockenem Methylenchlorid mit 0,7 g (0,016 Mol) Ameisensäure und 0,1 g Ammoniummolybdat versetzt. Unter starkem Rühren werden 2,3 g (0,024 Mol) 35%ige Wasserstoffperoxid-Lösung bei Raumtemperatur zugetropft. Die Mischung wird über 16 Stunden bei Raumtemperatur gerührt, die organische Phase abgetrennt und mit Natriumhydrogensulfit-Lösung gewaschen. Die erhaltene Lösung wird über Natriumsulfat getrocknet und eingeengt. Der Rückstand wird mit Diethylether verrührt, das erhaltene kristalline Produkt abgesaugt und getrocknet.

Man erhält 2,1 g (76 % der Theorie) an 2-(3-Chlorphenyl)-4-methylsulfonyl-6-(4-pyridyl)-pyridin vom Schmelzpunkt 217° C.

Analog zum Herstellungsbeispiel (III-1) und gemäß den allgemeinen Angaben zur Herstellung erhält man die in Tabelle 3 aufgeführten Verbindungen der allgemeinen Formel (III)

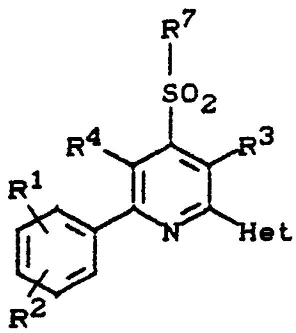

(III)

**Tabelle 3**

| Bsp. Nr. | $R^1$ | $R^2$ | $R^3$ | $R^4$ | $R^7$ | Het | Schmelzpunkt |
|---|---|---|---|---|---|---|---|
| III-2 | 4-Cl | H | H | H | $CH_3$ | 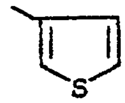 | 179° C |
| III-3 | 4-Cl | H | H | H | $CH_3$ | 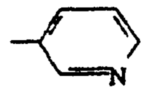 | 130° C (Zers.) |
| III-4 | 4-Cl | H | H | H | $CH_3$ | 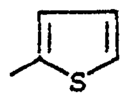 | 175° C |
| III-5 | $3-CF_3$ | H | H | H | $CH_3$ | 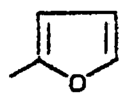 | 136° C |
| III-6 | $3-CF_3$ | H | H | H | $CH_3$ | 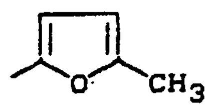 | 142° C (Zers.) |
| III-7 | $3-CF_3$ | H | H | H | $CH_3$ | 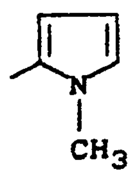 | 140° C |

<u>Tabelle 3</u> (Fortsetzung)

| Bsp. Nr. | $R^1$ | $R^2$ | $R^3$ | $R^4$ | $R^7$ | Het | Schmelzpunkt |
|---|---|---|---|---|---|---|---|
| III-8 | 3-$CF_3$ | H | H | H | $CH_3$ | 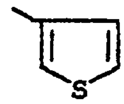 | 151° C |
| III-9 | 3-$CF_3$ | H | H | H | $CH_3$ | 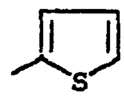 | 157° C |
| III-10 | 3-$CF_3$ | H | H | H | $CH_3$ | 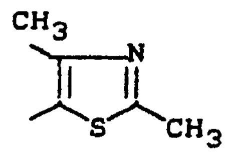 | 174° C |
| III-11 | H | H | H | H | $CH_3$ | 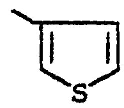 | 186° C |
| III-12 | H | H | H | H | $CH_3$ | 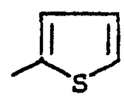 | 177° C |
| III-13 | H | H | H | H | $CH_3$ | 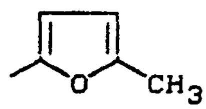 | 171° C |
| III-14 | H | H | H | H | $CH_3$ | 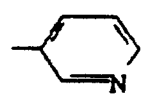 | 196° C |
| III-15 | 3-Cl | H | H | H | $CH_3$ | 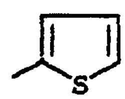 | 129° C |
| III-16 | 3-Cl | H | H | H | $CH_3$ | 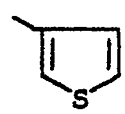 | 142° C |

Tabelle 3 (Fortsetzung)

| Bsp. Nr. | $R^1$ | $R^2$ | $R^3$ | $R^4$ | $R^7$ | Het | Schmelzpunkt |
|---|---|---|---|---|---|---|---|
| III-17 | 3-Cl | H | H | H | $CH_3$ | 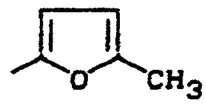 | 151° C |
| III-18 | 3-Cl | 5-Cl | H | H | $CH_3$ | 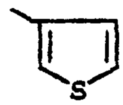 | 203° C |
| III-19 | 4-Cl | H | H | $CH_3$ | $CH_3$ | 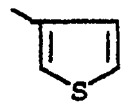 | 176° C |
| III-20 | $3-CF_3$ | $5-CF_3$ | H | H | $CH_3$ | 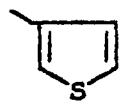 | 222° C |
| III-21 | 2-F | 4-F | H | H | $CH_3$ | 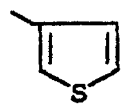 | 151° C |
| III-22 | 3-Cl | H | H | $CH_3$ | $CH_3$ | 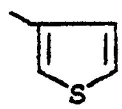 | 134° C |
| III-23 | H | H | H | $CH_3$ | $CH_3$ | 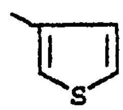 | 164° C |
| III-24 | H | H | H | $-C_2H_5$ | $CH_3$ | 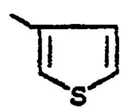 | 140° C (Zers.) |
| III-25 | 4-Cl | H | H | H | $CH_3$ | 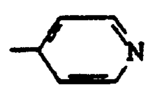 | 240° C |
| III-26 | 2-Cl | H | H | H | $CH_3$ | 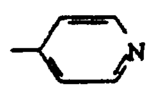 | 74° C |

Tabelle 3 (Fortsetzung)

| Bsp. Nr. | $R^1$ | $R^2$ | $R^3$ | $R^4$ | $R^7$ | Het | Schmelzpunkt |
|---|---|---|---|---|---|---|---|
| III-27 | 3-Cl | H | H | H | $CH_3$ | 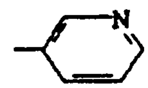 | 189° C |
| III-28 | 3-Cl | H | H | H | $CH_3$ | 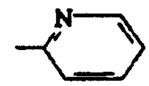 | 173° C |
| III-29 | 3-Br | H | H | H | $CH_3$ | 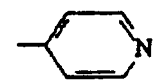 | 220° C |
| III-30 | $3-CF_3$ | H | H | H | $CH_3$ | 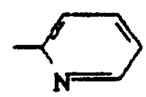 | 227° C |
| III-31 | $3-CF_3$ | H | H | H | $CH_3$ | 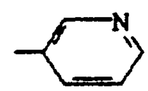 | 229° C |

Beispiel (IX-1)

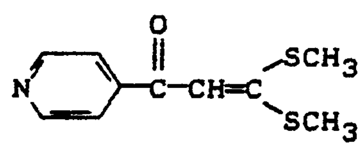

Zu 30,3 g (0,25 Mol) 4-Acetylpyridin in 300 ml trockenem Dimethylformamid werden 20,1 g (0,265 Mol) Schwefelkohlenstoff zugesetzt und dann portionsweise 16,5 g (0,55 Mol) Natriumhydrid (in Form einer 80%igen Suspension in Öl). Die Temperatur steigt dabei auf ca. 40° C an. Man rührt 30 Minuten bei Raumtemperatur und tropft dann 88,8 g (0,623 Mol) Methyliodid, während eines Zeitraumes von 30 Minuten, hinzu, Man rührt die Reaktionsmischung 3 Stunden bei Raumtemperatur und versetzt sie dann mit 600 ml Eiswasser. Der entstandene Niederschlag wird abgesaugt, mit Wasser gewaschen und aus Isopropanol umkristallisiert.
Man erhält 19,8 g (35 % der Theorie) an 3,3-Bis-(methylthio)-1-(4-pyridyl)-2-propen-1-on vom Schmelzpunkt 252° C (Zersetzung).

Beispiel (IX-2)

Analog zu Herstellungsbeispiel (IX-1) erhält man 3,3-Bis(methylthio)-1-(3-thienyl)-2-propen-1-on vom Schmelzpunkt 102° C.

Anwendungsbeispiele

Beispiel A

Post-emergence-Test (Gewächshaus)

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine sehr gute Wirksamkeit und Nutzpflanzenselektivität zeigen in diesem Test z.B. die Verbindungen gemäß folgender Herstellungsbeispiele; 2, 8, 23 und 39.

Beispiel B

Pre-emergence-Test (Gewächshaus)

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele 27, 39 und 2.

## Patentansprüche

1. 2-Aryl-6-hetarylpyridin-Derivate der allgemeinen Formel (I),

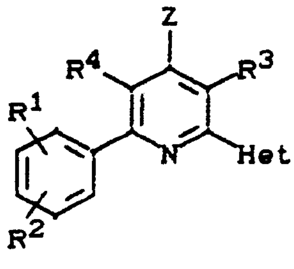

(I)

in welcher

| | |
|---|---|
| $R^1$ | für Wasserstoff, Halogen, Alkyl, Alkoxy oder Halogenalkyl steht, |
| $R^2$ | für Wasserstoff, Halogen oder Halogenalkyl steht, |
| $R^3$ und $R^4$ | unabhängig voneinander für Wasserstoff oder Alkyl stehen, |
| Het | für jeweils unsubstituiertes oder substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht und |
| Z | für Wasserstoff, Halogen oder die Gruppierungen $-X_n-R^{5-1}$ oder |

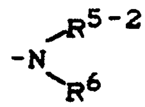

steht, wobei

| | |
|---|---|
| X | für Sauerstoff oder Schwefel steht, |
| $R^{5-1}$ und $R^{5-2}$ | unabhängig voneinander für jeweils gegebenenfalls substituiertes Alkyl, Alkenyl oder Alkinyl steht, |
| $R^6$ | für Wasserstoff oder Alkyl steht, oder |
| $R^{5-2}$ und $R^6$ | gemeinsam mit dem Stickstoffatom, an welches sie gebunden sind, einen 5- bis 7-gliedrigen gesättigten oder ungesättigten Ring bilden, welcher gegebenenfalls ein weiteres Heteroatom enthält und welcher gegebenenfalls durch 1 oder 2 Alkylgruppen substituiert ist, und |
| n | für 0 oder 1 steht, |

ausgenommen die Verbindungen 5-Methyl-3-trifluormethyl-1-(6-phenyl-4-trifluormethyl-2-pyridyl)-pyrazol, 2-(4-Dimethylamino-phenyl)-6-(2,5-dimethyl-pyrrol-1-yl)-pyridin. 6-Phenyl-(2,2'-bipyridyl), 4-Methylthio-6-phenyl-2,2'-bipyridin; 4-Methyl-2-phenyl-6-(2-thienyl)-pyridin, 6-[4-(Brommethyl)phenyl]-2,2'-bipyridin; 6-(4-Methylphenyl)-2,2'-bipyridin und 4-(Methylthio)-2-phenyl-6-(2-thienyl)-pyridin.

**2.** 2-Aryl-6-Hetarylpyridin-Derivate der Formel (I) gemäß Anspruch 1,

in welcher

| | |
|---|---|
| $R^1$ | für Wasserstoff, Halogen, jeweils geradkettiges oder verzweigtes Alkyl oder Alkoxy mit jeweils 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen steht, |
| $R^2$ | für Wasserstoff, Halogen, geradkettiges oder verzweigtes Halogenalkyl mit 1 bis 6 Kohlenstoffatomen und 1 bis 13 gleichen oder verschiedenen Halogenatomen steht, |
| $R^3$ und $R^4$ | unabhängig voneinander jeweils für Wasserstoff oder geradkettiges oder verzweigtes Alkyl mit 1 bis 6 Kohlenstoffatomen stehen, |
| Het | für jeweils unsubstituiertes oder einfach oder mehrfach, gleich oder verschieden substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht, wobei als Substituenten ausgewählt sind: |
| | Halogen, Cyano, Nitro, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Alkoxy oder Alkylthio mit jeweils 1 bis 6 Kohlenstoffatomen, jeweils geradkettiges oder verzweigtes Halogenalkylthio oder Halogenalkoxy mit jeweils 1 bis 6 Kohlenstoffatomen und jeweils 1 bis 13 gleichen oder verschiedenen Halogenatomen, Phenylamino, (Di)alkylamino mit 1 bis 6 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist, |
| Z | für Wasserstoff, Halogen oder die Gruppierungen $-X_n-R^{5-1}$oder |

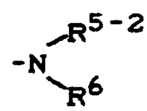

steht, wobei

X für Sauerstoff oder Schwefel steht,

$R^{5-1}$ und $R^{5-2}$ unabhängig voneinander für jeweils gegebenenfalls einfach oder mehrfach, gleich oder verschieden substituiertes, geradkettiges oder verzweigtes Alkyl mit 1 bis 8 Kohlenstoffatomen, Alkenyl mit 2 bis 6 Kohlenstoffatomen, Alkinyl mit 2 bis 6 Kohlenstoffatomen steht, wobei als Substituenten jeweils ausgewählt sind: Halogen, Cyano, geradkettiges oder verzweigtes Alkoxy mit 1 bis 6 Kohlenstoffatomen sowie geradkettiges oder verzweigtes Alkoxycarbonylalkyl mit 1 bis 6 Kohlenstoffatomen im Alkoxyteil und 1 bis 4 Kohlenstoffatomen im Alkylteil,

$R^6$ für Wasserstoff, geradkettiges oder verzweigtes Alkyl mit 1 bis 6 Kohlenstoffatomen steht, oder

$R^{5-2}$ und $R^6$ gemeinsam mit dem Stickstoffatom, an welches sie gebunden sind, einen 5- oder 6-gliedrigen gesättigten Ring bilden, welcher gegebenenfalls ein Sauerstoff-, Schwefel- oder Stickstoffatom enthält und welcher gegebenenfalls einfach oder zweifach durch Methyl und/oder Ethyl substituiert ist,

n für 0 oder 1 steht,

ausgenommen die Verbindungen 5-Methyl-3-trifluormethyl-1-(6-phenyl-4-trifluormethyl-2-pyridyl)-pyrazol, 2-(4-Dimethylamino-phenyl)-6-(2,5-dimethyl-pyrrol-1-yl)-pyridin, 6-Phenyl-(2,2'-bipyridyl), 4-Methylthio-6-phenyl-2,2'-bipyridin, 4-Methyl-2-phenyl-6-(2-thienyl)-pyridin, 6-[4-(Brommethyl)phenyl]-2,2'-bipyridin; 6-(4-Methylphenyl)-2,2'-bipyridin und 4-(Methylthio)-2-phenyl-6-(2-thienyl)-pyridin.

3. 2-Aryl-6-Hetarylpyridin-Derivate der Formel (I) gemäß Anspruch 1,

in welcher

$R^1$ für Wasserstoff, Fluor, Chlor, Brom, Methyl, Ethyl, n-Propyl, i-Propyl, n-Butyl- i-Butyl, s-Butyl, t-Butyl, Methoxy, Ethoxy, n-Propoxy, i-Propoxy, n-Butoxy, i-Butoxy, s-Butoxy, t-Butoxy oder für geradkettiges oder verzweigtes Halogenalkyl mit 1 bis 4 Kohlenstoffatomen und 1 bis 9 Fluor- und/oder Chloratomen steht,

$R^2$ für Wasserstoff, Fluor, Chlor, Brom, geradkettiges oder verzweigtes Halogenalkyl mit 1 bis 4 Kohlenstoffatomen und 1 bis 9 Fluor- und/oder Chloratomen steht,

$R^3$ und $R^4$ unabhängig voneinander jeweils für Wasserstoff, Methyl, Ethyl, n-Propyl, i-Propyl, n-Butyl, i-Butyl, s-Butyl oder t-Butyl stehen,

Het für jeweils unsubstituiertes oder 1- bis 3-fach, gleich oder verschieden substituiertes Pyridyl, Pyrazinyl, Pyrrolyl, Pyrazolyl, Imidazolyl, Thienyl, Furyl, Thiazolyl, Isothiazolyl, Oxazolyl oder Isoxazolyl steht, wobei als Substituenten jeweils ausgewählt sind: Fluor, Chlor, Brom, Iod, Cyano, Nitro, Methyl, Ethyl, n- oder i-Propyl, n-, i-, s-oder t-Butyl, jeweils geradkettiges oder verzweigtes Halogenalkyl, Halogenalkoxy oder Halogenalkylthio mit jeweils 1 bis 4 Kohlenstoffatomen und 1 bis 9 Fluor- und/oder Chloratomen, Phenylamino oder (Di)alkylamino mit 1 bis 4 Kohlenstoffatomen in den jeweiligen geradkettigen oder verzweigten Alkylteilen, wobei im Falle von Dialkylamino die beiden Alkylteile, gemeinsam mit dem Stickstoffatom, an welches sie gebunden sind, einen 5- oder 6-gliedrigen, gesättigten Ring bilden können, welcher gegebenenfalls ein Sauerstoff-, Schwefel- oder Stickstoffatom enthält und welcher gegebenenfalls durch 1 oder 2 Methylgruppen substituiert ist,

Z für Wasserstoff, Fluor, Chlor, Brom oder die Gruppierungen $-X_n-R^{5-1}$ oder

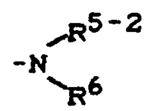

steht, wobei

X für Sauerstoff oder Schwefel steht,

$R^{5-1}$ und $R^{5-2}$ unabhängig voneinander für jeweils gegebenenfalls 1- oder 2-fach, gleich oder verschieden substituiertes Alkyl mit 1 bis 6 Kohlenstoffatomen, geradkettiges oder verzweigtes Alkenyl mit 2 bis 4 Kohlenstoffatomen, geradkettiges oder

verzweigtes Alkinyl mit 2 bis 4 Kohlenstoffatomen steht, wobei als Substituenten jeweils ausgewählt sind: Fluor, Chlor, Brom, Cyano, geradkettiges oder verzweigtes Alkoxycarbonylalkyl mit 1 bis 4 Kohlenstoffatomen im Alkoxyteil und 1 oder 2 Kohlenstoffatomen im Alkylteil,

$R^6$ für Wasserstoff, Methyl, Ethyl, n- oder i-Propyl, n-, i-, s- oder t-Butyl steht, oder

$R^{5-2}$ und $R^6$ gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylguppen substituiert ist,

n für 0 oder 1 steht,

ausgenommen die Verbindungen 5-Methyl-3-trifluormethyl-1-(6-phenyl-4-trifluormethyl-2-pyridyl)-pyrazol 2-(4-Dimethylamino-phenyl)-6-(2,5-dimethyl-pyrrol-1-yl)-pyridin, 6-Phenyl-(2,2'-bipyridyl); 4-Methylthio-6-phenyl-2,2'-bipyridin, 4-Methyl-2-phenyl-6-(2-thienyl)-pyridin, 6-[4-(Brommethyl)-phenyl]-2,2'-bipyridin, 6-(4-Methylphenyl)-2,2'-bipyridin, 4-(Methylthio)-2-phenyl-6-(2-thienyl)-pyridin.

4. Verfahren zur Herstellung von 2-Aryl-6-Hetarylpyridin-Derivaten der Formel (I)

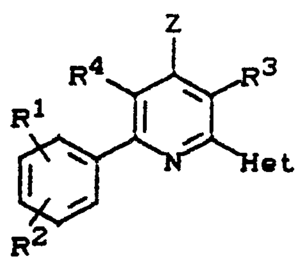

(I)

in welcher

$R^1$ für Wasserstoff, Halogen, Alkyl, Alkoxy oder Halogenalkyl steht,

$R^2$ für Wasserstoff, Halogen oder Halogenalkyl steht,

$R^3$ und $R^4$ unabhängig voneinander für Wasserstoff oder Alkyl stehen,

Het für einen unsubstituierten oder substituierten Heterocyclus steht und

Z für Wasserstoff, Halogen oder die Gruppierungen $-X_n-R^{5-1}$ oder

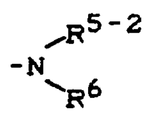

steht, wobei

X für Sauerstoff oder Schwefel steht,

$R^{5-1}$ und $R^{5-2}$ unabhängig voneinander für jeweils gegebenenfalls substituiertes Alkyl, Alkenyl oder Alkinyl steht,

$R^6$ für Wasserstoff oder Alkyl steht, oder

$R^{5-2}$ und $R^6$ gemeinsam mit dem Stickstoffatom, an welches sie gebunden sind, einen 5- bis 7-gliedrigen gesättigten oder ungesättigten Ring bilden, welcher gegebenenfalls ein weiteres Heteroatom enthält und welcher gegebenenfalls durch 1 oder 2 Alkylgruppen substituiert ist, und

n für 0 oder 1 steht,

ausgenommen die Verbindungen 5-Methyl-3-trifluormethyl-1-(6-phenyl-4-trifluormethyl-2-pyridyl)-pyrazol, 2-(4-Dimethylamino-phenyl)-6-(2,5-dimethyl-pyrrol-1-yl)-pyridin, 6-Phenyl-(2,2'-bipyridyl), 4-Methylthio-6-phenyl-2,2'-bipyridin; 4-Methyl-2-phenyl-6-(2-thienyl)-pyridin, 6-[4-(Brommethyl)phenyl]-2,2'-bipyridin; 6-(4-Methylphenyl)-2,2'-bipyridin und 4-(Methylthio)-2-phenyl-6-(2-thienyl)-pyridin,

dadurch gekennzeichnet, daß man

a) 2-Aryl-6-hetarylpyridin-Derivate der Formel (Ia)

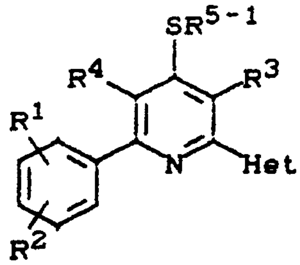

(Ia)

in welcher

$R^1$, $R^2$, $R^3$, $R^4$, $R^{5-1}$ und Het die oben angegebene Bedeutung haben,

erhält, wenn man 1,5-Dioxo-Verbindungen der Formel (II) bzw. deren Isomere der Formel (IIa)

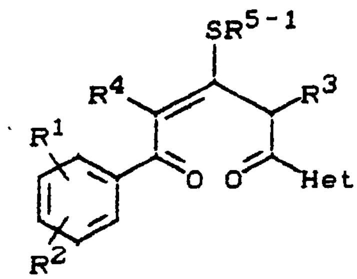

(II)

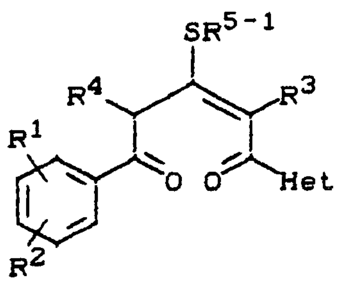

(IIa)

in welcher

$R^1$, $R^2$, $R^3$, $R^4$, $R^{5-1}$ und Het die oben angegebene Bedeutung haben,

mit Ammoniak oder einem Ammoniumsalz gegebenenfalls in Gegenwart eines Verdünnungsmittels umsetzt, oder daß man

b) 2-Aryl-6-hetarylpyridin-Derivate der Formel (Ib)

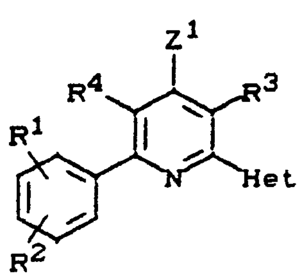

(Ib)

in welcher

$Z^1$ für $-OR^{5-1}$ oder

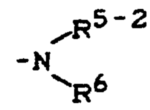

steht und

$R^1$, $R^2$, $R^3$, $R^4$, $R^{5-1}$, $R^{5-2}$, $R^6$ und Het   die oben angegebene Bedeutung haben,

erhält, wenn man 4-Sulfonylalkyl-2-aryl-6-hetarylpyridin-Derivate der Formel (III)

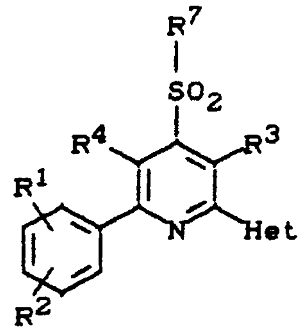

(III)

in welcher

$R^1$, $R^2$, $R^3$, $R^4$ und Het   die oben angegebene Bedeutung haben und

$R^7$   für Alkyl steht, entweder

α) mit Verbindungen der Formel (IV)

$R^{5-1}$-OM(IV)

in welcher

$R^{5-1}$   die oben angegebene Bedeutung hat und

M   für ein Alkalimetallkation steht,

gegebenenfalls in Gegenwart eines Verdünnungsmittels umsetzt, oder

ß) mit Aminen der Formel (V)

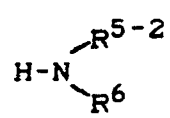

(V)

in welcher

$R^{5-2}$ und $R^6$   die oben angegebene Bedeutung haben,

gegebenenfalls in Gegenwart eines Verdünnungsmittels und gegebenenfalls unter erhöhtem Druck umsetzt, oder daß man

c) 2-Aryl-6-hetarylpyridin-Derivate der Formel (Ic)

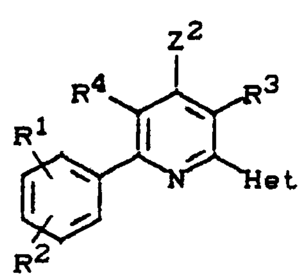

(Ic)

in welcher
$R^1$, $R^2$, $R^3$, $R^4$ und Het die oben angegebene Bedeutung haben und
$Z^2$ für Halogen steht,
erhält, wenn man 4-Hydroxy-2-aryl-6-hetarylpyridin-Derivate der Formel (VI)

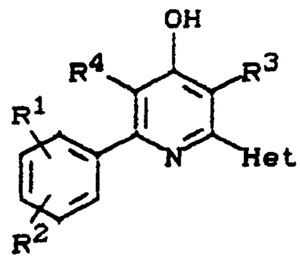

(VI)

in welcher
$R^1$, $R^2$, $R^3$, $R^4$ und Het die oben angegebene Bedeutung haben,
mit einem Halogenierungsreagenz gegebenenfalls in Gegenwart eines Verdünnungsmittels umsetzt, oder daß man
d) 2-Aryl-6-hetarylpyridin-Derivate der Formel (Id)

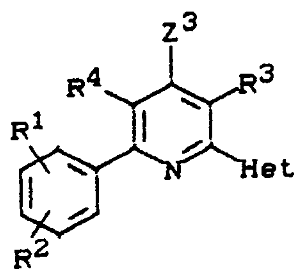

(Id)

in welcher
$R^1$, $R^2$, $R^3$, $R^4$ und Het die oben angegebene Bedeutung haben, und
$Z^3$ für Wasserstoff, jeweils gegebenenfalls substituiertes Alkyl, Alkenyl oder Alkinyl steht,
erhält, wenn man 2-Aryl-6-hetarylpyridin-Derivate der Formel (Ic)

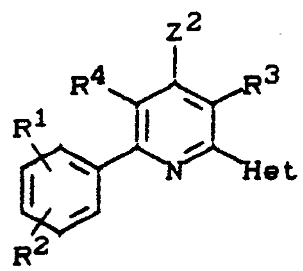

(Ic)

in welcher
$R^1$, $R^2$, $R^3$, $R^4$ und Het die oben angegebene Bedeutung haben, und
$Z^2$ für Halogen steht,
α) entweder gegebenenfalls in Gegenwart eines Verdünnungsmittels, gegebenenfalls in Gegenwart einer Base, in Gegenwart eines Katalysators und unter Wasserstoffdruck enthalogeniert, oder
β) mit Diester-Derivaten der Formel (VII)

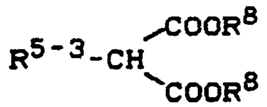

(VII)

in welcher

$R^{5-3}$ für Wasserstoff oder jeweils gegebenenfalls substituiertes Alkyl, Alkenyl oder Alkinyl steht, und

$R^8$ für Methyl oder Ethyl steht,

gegebenenfalls in Gegenwart eines Verdünnungsmittels und gegebenenfalls in Gegenwart einer Base umsetzt und die so erhaltenen Verbindungen gegebenenfalls in Gegenwart eines Verdünnungsmittels und in Gegenwart einer Base hydrolysiert und anschließend in Gegenwart einer Säure decarboxyliert.

5. Herbizide Mittel, gekennzeichnet durch einen Gehalt an mindestens einem 2-Aryl-6-hetarylpyridin-Derivat der Formel (I) gemäß Anspruch 1 oder 4.

6. Verfahren zur Bekämpfung von unerwünschten Pflanzen, dadurch gekennzeichnet, daß man 2-Aryl-6-hetarylpyridin-Derivate der Formel (I) gemäß Anspruch 1 oder 4 auf die Planzen und/oder ihren Lebensraum einwirken läßt.

7. Verwendung von 2-Aryl-6-hetarylpyridin-Derivaten der Formel (I) gemäß Anspruch 1 oder 4 zur Bekämpfung von unerwünschten Pflanzen.

8. Verfahren zur Herstellung von herbiziden Mitteln, dadurch gekennzeichnet, daß man 2-Aryl-6-hetarylpyridin-Derivate der Formel (I) gemäß Anspruch 1 oder 4 mit Streckmitteln und/oder oberflächenaktiven Substanzen vermischt.

9. 4-Sulfonylalkyl-2-aryl-6-hetarylpyridin-Derivate der Formel (III)

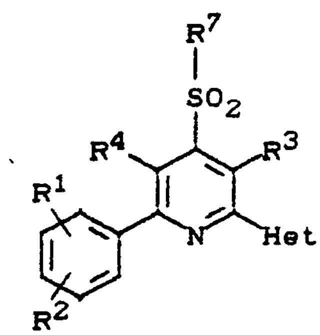

(III)

in welcher

$R^1$ für Wasserstoff, Halogen, Alkyl, Alkoxy oder Halogenalkyl steht,

$R^2$ für Wasserstoff, Halogen oder Halogenalkyl steht,

$R^3$ und $R^4$ unabhängig voneinander für Wasserstoff oder Alkyl stehen,

Het für einen unsubstituierten oder substituierten Heterocyclus steht und

$R^7$ für Alkyl steht.

10. 4-Hydroxy-2-aryl-6-hetarylpyridin-Derivate der Formel (VI)

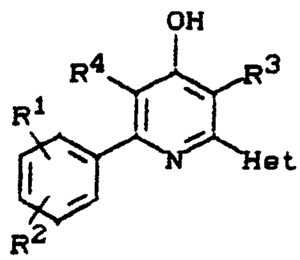

(VI)

in welcher

| | |
|---|---|
| $R^1$ | für Wasserstoff, Halogen, Alkyl, Alkoxy oder Halogenalkyl steht, |
| $R^2$ | für Wasserstoff, Halogen oder Halogenalkyl steht, |
| $R^3$ und $R^4$ | unabhängig voneinander für Wasserstoff oder Alkyl stehen und |
| Het | für einen unsubstituierten oder substituierten Heterocyclus steht. |